# EUROPÄISCHE PATENTSCHRIFT

(11) **EP 3 478 653 B1**
(45) Veröffentlichungstag und Bekanntmachung des Hinweises auf die Patenterteilung: **19.02.2020**
(21) Anmeldenummer: 17732926.5
(22) Anmeldetag: 27.06.2017
(51) Int. Cl.: C07C 209/68

(54) **VERFAHREN ZUR HERSTELLUNG VON ANILIN ODER EINES ANILINFOLGEPRODUKTS**
METHOD FOR THE PREPARATION OF ANILINE OR AN ANILINE DERIVATIVE
PROCÉDÉ DE FABRICATION D'ANILINE OU D'UN DÉRIVE D'ANILINE

(30) Priorität: 29.06.2016 EP 16177000
(43) Veröffentlichungstag der Anmeldung: 08.05.2019
(73) Patentinhaber: Covestro Deutschland AG, 51373 Leverkusen (DE)
(72) Erfinder: JÄGER, Gernot, 50733 Köln (DE); HAMEDINGER, Thomas, 51375 Leverkusen (DE); LOLLI, Giulio, 51069 Köln (DE); MOUSSA, Amgad, Salah, 79713 Bad Säckingen (DE); OLF, Guenter, 53909 Zülpich (DE)
(74) Vertreter: Levpat
(86) Internationale Anmeldenummer: PCT/EP2017/065913
(87) Internationale Veröffentlichungsnummer: WO 2018/002088

(56) Entgegenhaltungen:
- WO-A1-2015/124686

## Beschreibung

Die vorliegende Erfindung befasst sich mit einem Verfahren zur Herstellung von Anilin oder Produkten, die durch weitere chemische Umsetzung von Anilin erhalten werden (im Folgenden als "Anilinfolgeprodukte" oder "Anilinderivate" bezeichnet; beide Begriffe gelten im Rahmen der vorliegenden Erfindung als gleichbedeutend), umfassend eine Decarboxylierung von Aminobenzoesäure, insbesondere ortho-Aminobenzoesäure, wobei im Decarboxylierungsschritt ein Teil des zuvor gebildeten rohen Anilins zurückgeführt wird. Die Aminobenzoesäure wird fermentativ oder chemisch, bevorzugt fermentativ, gewonnen.

Die Herstellung von Anilin durch Decarboxylierung von Aminobenzoesäure ist grundsätzlich im Stand der Technik bekannt; siehe beispielsweise Per Wiklund et al., Current Organic Synthesis, 2006, 3, 379 - 402. In Stevens et al., Canadian Journal of Chemistry, 1952, 30 (7), 529 - 540, wird berichtet, dass eine wässrige Lösung von ortho-Aminobenzoesäure in Gegenwart von 0,75 N Schwefelsäure in 6 Stunden bei 100 °C mit 56%iger Ausbeute zu Anilin decarboxyliert werden konnte. In MacMaster and Shriner, J. Am. Chem. Soc., 1923, 45 (3), 751 - 753 war zuvor berichtet worden, dass unter ähnlichen Bedingungen (in siedendem Wasser) aber in Abwesenheit von Säure ortho-Aminobenzoesäure in 7 Stunden mit nur 27%iger Ausbeute zu Anilin decarboxyliert wurde.

Auch in der jüngeren Patentliteratur finden sich hierzu Veröffentlichungen; siehe beispielsweise WO 2015/124686 A1 und WO 2015/124687 A1. In WO 2015/124686 A1 wird die thermische Decarboxylierung von ortho-Aminobenzoesäure in Gegenwart von oder ohne Katalysator in wässriger Umgebung beschrieben. In WO 2015/124687 A1 wird die katalytische Decarboxylierung durch Zeolith-Katalyse in 1-Decanol als Lösungsmittel beschrieben. Beide Anmeldungen beschreiben ferner die weitere Umsetzung des so hergestellten Anilins zu Anilinderivaten wie den Di- und Polyaminen der Diphenylmethanreihe und den korrespondierenden Isocyanaten.

Die Ausgangsverbindung Aminobenzoesäure kann chemisch oder vorzugsweise fermentativ gewonnen werden.

Die *chemische* Herstellung von Aminobenzoesäure ist in der Literatur beschrieben. Eine geeignete Syntheseroute (mit Ausbeuten > 98 %) ist beispielsweise die Reaktion von Phthalimid mit Natriumhypochlorit. Phthalimid kann seinerseits aus Phthalsäureanhydrid und Ammoniak gewonnen werden. Der ganze Prozess ist wohlbekannt und wird z. B. in Lorz et al., Phthalic Acid and Derivatives in Ullmann's Encyclopedia of Industrial Chemistry, Band 27, S. 140 - 141, Weinheim, Wiley-VCH, beschrieben. Ein industrieller Prozess ist ebenfalls in der Patentliteratur beschrieben; siehe z. B. DE 29 02 978 A1 und EP 0 004 635 A2.

Die *fermentative* Herstellung von Aminobenzoesäure ist in der Literatur beschrieben. Für die fermentative Herstellung von Aminobenzoesäure sei beispielhaft auf Balderas-Hemandez, V. E. et al., "Metabolic engineering for improving anthranilate synthesis from glucose in Escherichia coli", Microb. Cell. Fact. 2009, 8, 19 (doi: 10.118611475-2859-8-19) verwiesen. Auch in der Patentliteratur finden sich hierzu Veröffentlichungen; siehe beispielsweise die bereits erwähnten Anmeldungen WO 2015/124686 A1 und WO 2015/124687 A1 sowie die darin jeweils zitierte Literatur.

Fermentationsprozesse laufen in der Regel in einer wässrigen Umgebung ab und liefern im Falle der Herstellung von Aminobenzoesäure im Allgemeinen wässrige Lösungen (Fermentationsbrühen) mit einem Massengehalt an Aminobenzoesäure im Bereich von 10,0 g/L bis 100 g/L. Der in WO 2015/124686 A1 beschriebene Ansatz, die wässrige Lösung von ortho-Aminobenzoesäure, gegebenenfalls nach Abtrennung von Biomasse, direkt zu decarboxylieren, ist durchaus nicht per se unattraktiv. Allerdings erfordert das in WO 2015/124686 A1 beschriebene Verfahren die Extraktion von in der Decarboxylierung gebildetem Anilin mit einem systemfremden organischen Lösungsmittel (ein Alkohol, Phenol, Amid, Ether oder aromatischer Kohlenwasserstoff; insbesondere wird 1-Dodecanol als geeignetes Lösungsmittel hervorgehoben), was unvermeidbar mit zusätzlichen Kosten und zusätzlichem Reinigungsaufwand (Trennung Anilin von 1-Dodecanol) verbunden ist.

WO 2015/124687 A1 beschreibt die *Durchführung der Decarboxylierung* u. a. in Wasser oder in einem systemfremden organischen Lösungsmittel, insbesondere 1-Dodecanol, gegebenenfalls im Gemisch mit Anilin (vgl. Seite 18, Zeilen 28 und 29). Die zuvor geschilderten Nachteile des Einsatzes systemfremder organischer Lösungsmittel sind daher auch für diese Ausführungsformen der Decarboxylierung relevant. Darüber hinaus beschreibt diese Schrift auch die Möglichkeit der Durchführung der Decarboxylierung in Anilin (ohne 1-Dodecanol; siehe die Abbildungen 35 und 37 bis 38 und die zugehörigen Textstellen), gegebenenfalls in Gegenwart von 10 Massen-% Wasser (siehe die Abbildung 36 und die zugehörigen Textstellen). Die Schrift macht zwar keine explizite Angabe zur Herkunft des verwandten Anilins; aus dem Zusammenhang ist für den Fachmann jedoch offensichtlich, dass es sich um Reinanilin handelte. Die Beschreibung dieser Verfahrensvariante geht aber über das Aufzeigen der grundsätzlichen Möglichkeit einer solchen Decarboxylierung von Aminobenzoesäure aus unterschiedlichen Quellen in Anilin nicht hinaus. Verfahrenstechnische Details zur Quelle und Ausgestaltung der Zuführung des im Decarboxylierungsschritt *einzusetzenden* Anilins in einen vorzugsweise kontinuierlich zu betreibenden großtechnischen Prozess sind der Schrift nicht zu entnehmen.

Weitere Verbesserungen in der Herstellung von Anilin bzw. Anilinfolgeprodukten durch Decarboxylierung von, insbesondere fermentativ gewonnener, Aminobenzoesäure wären daher wünschenswert. Insbesondere wäre es wünschenswert, das Verfahren möglichst einfach und ohne den Einsatz systemfremder Lösungsmittel (wie 1-Dodecanol) ausgestalten zu können, um die Wirtschaftlichkeit des Verfahrens zu erhöhen und so dessen Einsatz im großtechnischen Produktionsmaßstab attraktiver zu machen. Weiterhin wäre es wünschenswert, Verbesserungen im Decarboxylierungsschritt so auszugestalten, dass die der Decarboxylierung folgende, bevorzugt destillativ durchgeführte, Aufreinigung des gewonnenen Anilins nicht erschwert oder sogar vereinfacht wird.

Dem vorstehend Gesagten Rechnung tragend ist ein Gegenstand der vorliegenden Erfindung ein **Verfahren zur Herstellung von Anilin oder eines Anilinfolgeprodukts,** umfassend die folgenden Schritte:
(I) Decarboxylierung von Aminobenzoesäure, insbesondere ortho-Aminobenzoesäure, zu Anilin in einem Reaktor in Gegenwart eines Katalysators, wobei ein Anilin enthaltender Strom (nachfolgend auch "Rohanilin" genannt) dem Reaktor entnommen wird;
(II) Aufreinigung eines Teils des in Schritt (I) entnommenen, Anilin enthaltenden Stroms zur Gewinnung von Anilin, bevorzugt durch Destillation;
(III) Rückführung eines anderen Teils des in Schritt (I) entnommenen, Anilin enthaltenden Stroms (nachfolgend auch "Rückführanilin" genannt) in den Reaktor aus Schritt (I);
(IV) optionale weitere Umsetzung des in Schritt (II) aufgereinigten Anilins zu einem Anilinfolgeprodukt.

Vollkommen überraschend wurde gefunden, dass die Rückführung eines Teils des in Schritt (I) entnommenen, Anilin enthaltenden Stroms in den Reaktor aus Schritt (I) trotz der damit unweigerlich verbundenen Rückführung auch von potenziell katalysatorschädlichen Nebenprodukten erfolgreich durchgeführt werden kann und so die Zugabe systemfremder organischer Lösungsmittel überflüssig wird. (Im Rahmen der vorliegenden Erfindung werden als "*systemfremde* organische Lösungsmittel" solche organischen Lösungsmittel verstanden, die *nicht* verfahrensimmanent sind, also nicht zwangsläufig ohnehin im Verfahren zugegen sind. Anilin, welches in dem im Rahmen von Schritt (III) in den Reaktor aus Schritt (I) zurückzuführenden Strom enthalten ist, ist in diesem Sinne als *verfahrensimmanentes* Lösungsmittel aufzufassen. Im Sinne dieser Erfindung wird ferner das in bevorzugten Ausführungsformen optional aus einer *externen* Quelle *zusätzlich* dem Reaktor aus Schritt (I) zugeführte Anilin ebenfalls als *verfahrensimmanentes* Lösungsmittel verstanden, weil Anilin das Produkt des Verfahrens ist.) Es hat sich nämlich überraschend herausgestellt, dass durch die Rückführung eines Teils des Rohanilins ein für die erfolgreiche Durchführung der Reaktion vollkommen ausreichendes und zufriedenstellende Resultate lieferndes Lösungsmittel zur Verfügung steht.

Der Begriff "Anilinfolgeprodukt" bezeichnet im Rahmen der vorliegenden Erfindung ein Produkt, das durch weitere chemische Umwandlung von Anilin erhalten wird.

Es folgt zunächst eine Kurzzusammenfassung verschiedener möglicher **Ausführungsformen der Erfindung:**
In einer **ersten Ausführungsform** der Erfindung, die mit allen anderen Ausführungsformen kombiniert werden kann, wird dem Reaktor aus Schritt (I) zusätzlich zu dem in Schritt (III) zurückgeführten Anilin enthaltenden Strom ein weiterer Anilinstrom aus gereinigtem, vorzugsweise destilliertem Anilin, insbesondere aus Anilin mit einem per Gaschromatographie bestimmten Anilingehalt von mindestens 99,00 Massen-%, bevorzugt mindestens 99,50 Massen-%, ganz besonders bevorzugt mindestens 99,90 Massen-% bezogen auf die Gesamtmasse des zusätzlich zugeführten Anilinstroms, zugeführt, wobei das auf diese Weise dem Reaktor aus Schritt (I) zusätzlich zugeführte Anilin maximal 60 %, bevorzugt von 1,0 % bis 50 %, bevorzugt 5,0 % bis 20 %, des dem Reaktor aus Schritt (I) insgesamt zugeführten Anilins ausmacht.

In einer **zweiten Ausführungsform** der Erfindung, die eine besondere Ausgestaltung der ersten Ausführungsform ist, wird die Konzentration an Anilin in dem Anilin enthaltenden Strom, der dem Reaktor aus Schritt (I) entnommen wird, überwacht, und bei festgestellter Unterschreitung eines zuvor festgelegten Werts der Konzentration an Anilin wird der Anteil des dem Reaktor zusätzlich zugeführten Anilinstroms aus gereinigtem, vorzugsweise destilliertem Anilin, insbesondere aus Anilin mit einem per Gaschromatographie bestimmten Anilingehalt von mindestens 99,00 Massen-%, bevorzugt mindestens 99,50 Massen-%, ganz besonders bevorzugt mindestens 99,90 Massen-% bezogen auf die Gesamtmasse des zusätzlich zugeführten Anilinstroms, auf einen Wert von maximal 60 % des dem Reaktor aus Schritt (I) insgesamt zugeführten Anilins erhöht.

In einer **dritten Ausführungsform** der Erfindung, die mit allen anderen Ausführungsformen kombiniert werden kann, wird der dem Reaktor aus Schritt (I) entnommene, Anilin enthaltende Strom in einem Massen-Verhältnis im Bereich von 9,0 : 1 bis 1 : 9,0, bevorzugt im Bereich von 1,5 : 1 bis 1 : 1,5, in zwei Ströme aufgeteilt, von denen einer, bevorzugt der Größere, der Rückführung aus Schritt (III) und der andere der Aufreinigung aus Schritt (II) zugeführt wird.

In einer **vierten Ausführungsform** der Erfindung, die mit allen anderen Ausführungsformen kombiniert werden kann, wird Schritt (I) bei einer Temperatur im Bereich von 140 °C bis 240 °C und bei einem absoluten Druck im Bereich von 1,00 bar bis 20,0 bar, bevorzugt bei einer Temperatur im Bereich von 160 °C bis 220 °C und bei einem absoluten Druck im Bereich von 1,00 bar bis 15,0 bar, besonders bevorzugt bei einer Temperatur im Bereich von 180 °C bis 200 °C und bei einem absoluten Druck im Bereich von 4,00 bar bis 10,0 bar, durchgeführt.

In einer **fünften Ausführungsform** der Erfindung, die mit allen anderen Ausführungsformen kombiniert werden kann, ist der Reaktor aus Schritt (I)
ein Schlammphasenreaktor, wobei der Katalysator in einer Konzentration im Bereich von 0,100 Massen-% bis 50,0 Massen-%, bevorzugt im Bereich von 10,0 Massen-% bis 30,0 Massen-%, bezogen auf die Gesamtmasse der flüssigen Reaktionsmischung, eingesetzt wird,
oder
ein Rührkesselreaktor,
oder
ein Rohrreaktor mit einem Katalysatorbett, wobei der Katalysator insbesondere in Partikeln vorliegt und vorzugsweise im Katalysatorbett fixiert ist.

In einer **sechsten Ausführungsform** der Erfindung, die mit allen anderen Ausführungsformen kombiniert werden kann, ist der in Schritt (I) eingesetzte Katalysator ein Zeolith-Katalysator, bevorzugt ein Zeolith vom Typ Y in der protonierten Form.

In einer **siebten Ausführungsform** der Erfindung, die mit allen anderen Ausführungsformen kombiniert werden kann, wird die in Schritt (I) zu decarboxylierende Aminobenzoesäure durch folgenden, vor Schritt (I) durchzuführenden Schritt (I-0) bereitgestellt:
(I-0) Fermentation eines Rohstoffes, der wenigstens
- eine fermentierbare Kohlenstoff-haltige Verbindung bevorzugt ausgewählt aus der Gruppe bestehend aus Stärkehydrolysat, Zuckerrohrsaft, Zuckerrübensaft und Hydrolysaten aus lignocellulosehaltigen Rohstoffen, und
- eine Stickstoff-haltige Verbindung, bevorzugt ausgewählt aus der Gruppe bestehend aus Ammonikgas, Ammoniakwasser, Ammoniumsalzen (insbesondere Ammoniumsulfat und Ammoniumchlorid) und Harnstoff,
umfasst,
in einem Fermentationsreaktor unter Verwendung von Mikroorganismen unter Erhalt einer Fermentationsbrühe; woran sich optional die folgenden Aufarbeitungsschritte anschließen:
(α) Abtrennung des Mikroorganismus aus der Fermentationsbrühe und/oder
(β) Entfärbung der Fermentationsbrühe oder, bei Durchführung von Schritt (a), der in Schritt (a) erhaltenen an Mikroorganismen abgereicherten Fermentationsbrühe.

In einer **achten Ausführungsform** der Erfindung, die eine besondere Ausgestaltung der siebten Ausführungsform ist, wird nach Schritt (I-0) und vor Schritt (I) folgender Schritt durchgeführt:
(I-0) (a) Anreicherung der Aminobenzoesäure durch eine der folgenden Maßnahmen:
(1) Eindampfen der Fermentationsbrühe, oder
(2) Fällung durch Säurebehandlung in Verbindung mit mindestens teilweiser Abtrennung der abgeschiedenen Aminobenzoesäure von der säurebehandelten Fermentationsbrühe.

In einer **neunten Ausführungsform** der Erfindung, die eine besondere Ausgestaltung der achten Ausführungsform ist, wird Schritt (I-0) (a) gemäß Variante (2) durchgeführt und umfasst Folgendes:
(i) Behandlung, bevorzugt einstufige Behandlung, der in Schritt (I-0), gegebenenfalls nach Durchführung von Schritt (α) und/oder Schritt (β), erhaltenen Fermentationsbrühe in einem Reaktor mit Säure so, dass sich Aminobenzoesäure aus der Fermentationsbrühe abscheidet, wobei bevorzugt der pH-Wert der resultierenden Mischung auf einen Wert im Bereich von 3,0 bis 4,7, bevorzugt im Bereich von 3,2 bis 3,7, besonders bevorzugt im Bereich von 3,4 bis 3,6, eingestellt wird;
(ii) mindestens teilweise Abtrennung der in Schritt (I-0) (a) (i) abgeschiedenen Aminobenzoesäure von der säurebehandelten Fermentationsbrühe;
(iii) optionale weitere Reinigung der in Schritt (I-0) (a) (ii) gewonnenen Aminobenzoesäure, bevorzugt durch Waschen mit Wasser.

In einer **zehnten Ausführungsform** der Erfindung, die eine besondere Ausgestaltung der neunten Ausführungsform ist, umfasst die in Schritt (I-0) (a) (i) eingesetzte Säure Salzsäure, Schwefelsäure und/oder Phosphorsäure, wobei die in Schritt (I-0) (a) (i) eingesetzte Säure bevorzugt Salzsäure einer Konzentration von 15 Massen-% bis 37 Massen-% umfasst, und besonders bevorzugt neben dieser Salzsäure mit Ausnahme von optional zugesetzter rezyklierter säurebehandelter Fermentationsbrühe aus Schritt (I-0) (a) (ii) keine weitere Säure umfasst.

In einer **elften Ausführungsform** der Erfindung, die eine besondere Ausgestaltung der siebten, achten, neunten oder zehnten Ausführungsform ist, umfassen die in Schritt (I-0) eingesetzten Mikroorganismen eine Art ausgewählt aus der Gruppe bestehend aus *Escherichia coli, Pseudomonas putida, Corynebacterium glutamicum, Ashbya gossypii, Pichia pastoris, Hansenula polymorpha, Yarrowia lipolytica, Zygosaccharomyces bailii und Saccharomyces cerevisiae,* und bestehen bevorzugt nur aus Vertretern genau einer dieser Arten, wobei *Corynebacterium glutamicum* ATTC 13032 ganz besonders bevorzugt ist.

In einer **zwölften Ausführungsform** der Erfindung, die mit allen anderen Ausführungsformen kombiniert werden kann, insbesondere mit der siebten, achten, neunten, zehnten oder elften Ausführungsform, wird der in Schritt (III) in den Reaktor aus Schritt (I) zurückgeführte, Anilin enthaltende Strom mit fester oder gelöster oder suspendierter Aminobenzoesäure versetzt, und zwar bevorzugt in einer solchen Menge, dass der Massenstrom dieser zugeführten festen oder gelösten oder suspendierten Aminobenzoesäure dem Massenstrom des in Schritt (II) der Aufreinigung zugeführten Teils des in Schritt (I) entnommenen, Anilin enthaltenden Stroms entspricht.

In einer **dreizehnten Ausführungsform** der Erfindung, die eine besondere Ausgestaltung der zwölften Ausführungsform in Verbindung mit der achten, neunten, zehnten oder elften Ausführungsform ist, wird der in Schritt (III) in den Reaktor aus Schritt (I) zurückgeführte, Anilin enthaltende Strom mit fester oder gelöster oder suspendierter Aminobenzoesäure versetzt, wobei diese aus Schritt (I-0) (a), insbesondere aus Schritt (I-0) (a) (ii) oder aus Schritt (I-0) (a) (iii) stammt und wasserhaltig ist, und wobei vorzugsweise der Wassergehalt und die Menge dieser festen oder gelösten oder suspendierten Aminobenzoesäure so eingestellt werden, dass der Wassergehalt der flüssigen Reaktionsmischung in Schritt (I) im Bereich von 0,10 Massen-% bis 40 Massen-%, bevorzugt 0,15 Massen-% bis 20 Massen-%, bezogen auf die Gesamtmasse der flüssigen Reaktionsmischung aus Schritt (I), liegt.

In einer **vierzehnten Ausführungsform** der Erfindung, die mit einer jeder der ersten bis elften Ausführungsform kombiniert werden kann, und insbesondere mit der siebten, achten, neunten, zehnten oder elften Ausführungsform kombiniert werden kann, werden der in Schritt (III) zurückgeführte, Anilin enthaltende Strom und die zu decarboxylierende Aminobenzoesäure dem Reaktor aus Schritt (I) über getrennte Zuführeinrichtungen zugeführt.

Die zuvor kurz geschilderten Ausführungsformen und weitere Ausgestaltungen der Erfindung werden nachfolgend näher beschrieben. Dabei können verschiedene Ausführungsformen beliebig miteinander kombiniert werden, sofern sich für den Fachmann aus dem Gesamtzusammenhang nicht das Gegenteil ergibt.

Aminobenzoesäure kommt in drei *isomeren Formen* (ortho-, meta- und para-Aminobenzoesäure) vor. Grundsätzlich kann das erfindungsgemäße Verfahren auf alle drei Isomere, entweder in isomerenreiner Form oder als Mischungen verschiedener Isomere, angewandt werden. Für alle Ausführungsformen der vorliegenden Erfindung gilt, dass die in **Schritt (I)** zu decarboxylierende Aminobenzoesäure vorzugsweise das ortho-Isomer umfasst. Besonders bevorzugt umfasst die in Schritt (I) zu decarboxylierende Aminobenzoesäure mindestens 50,0 mol-%, ganz besonders bevorzugt mindestens 90,0 mol-%, bezogen auf die Gesamtstoffmenge aller vorhandenen Isomere an Aminobenzoesäure, des ortho-Isomers. Außerordentlich ganz besonders bevorzugt besteht die in Schritt (I) zu decarboxylierende Aminobenzoesäure aus dem ortho-Isomer in isomerenreiner (d. h. Isomerenreinheit > 99,0 mol-%) Form.

Als *Reaktoren* für die Durchführung von Schritt (I) eignen sich grundsätzlich in der Verfahrenstechnik übliche, dem Fachmann geläufige Reaktortypen wie bspw. Rührkesselreaktoren (bevorzugt mit Katalysatorfestbett), kontinuierlich betriebene Rührkesselreaktoren (in der englischen Literatur als *continuous stirred tank reactors* (CSTR) bezeichnet), insbesondere kontinuierlich betriebene Rührkesselreaktoren (CSTR) mit Katalysatorfestbett, Kolbenstromreaktoren (in der englischen Literatur als *plug flow reactors* bezeichnet) mit Katalysatorfestbett oder Schlammphasenreaktoren (auch Suspensionsreaktoren genannt; in der englischen Literatur als *slurry phase reactors* bezeichnet) mit Katalysator-Rezirkulation bzw. Katalysator-Rückgewinnung.

Der Ausdruck *"in einem Reaktor"* umfasst erfindungsgemäß auch Ausführungsformen, in denen mehrere Reaktoren zu einer Reaktorkaskade hintereinandergeschaltet sind, d. h. der flüssige Produktaustrag eines Reaktors fließt zur weiteren Umsatzvervollständigung in den nächstfolgenden Reaktor. Es ist möglich, nur in den ersten Reaktor einer Reaktorkaskade die Edukte (also die zu decarboxylierende Aminobenzoesäure und zurückgeführtes Rohanilin) einzuspeisen. Es ist jedoch ebenfalls möglich, in jeden Reaktor einer Reaktorkaskade die zu decarboxylierende Aminobenzoesäure und zurückgeführtes Rohanilin einzuspeisen. Der Anilin enthaltende Strom, welcher den Schritten (II) und (III) zugeführt wird, wird dem letzten Reaktor der Reaktorkaskade entnommen.

Als *Katalysatoren* für die Durchführung von Schritt (I) eignen sich dem Fachmann geläufige Katalysatoren wie bspw. wässrige Säuren wie Schwefelsäure, Salpetersäure und Salzsäure; feste Säuren wie Zeolithe und Si-Ti-Molekularsiebe, feste Basen wie Hydroxy-Apatite und Hydrotalcite; polymere Säuren wie Ionenaustauscherharze (insbesondere Amberlyst). Wird der Katalysator in Form von Partikeln oder in Pulverform eingesetzt, so besteht eine bevorzugte Ausführungsform der Erfindung darin, den Katalysator in der flüssigen Reaktionsmischung aufzuschlämmen, bevorzugt durch Rühren. Hierzu eignet sich besonders ein Schlammphasenreaktor (auch Suspensionsreaktor genannt), wobei der Katalysator in einer Konzentration im Bereich von 0,100 Massen-% bis 50,0 Massen-%, bevorzugt im Bereich von 10,0 Massen-% bis 30,0 Massen-%, bezogen auf die Gesamtmasse der flüssigen Reaktionsmischung, eingesetzt wird. In einer anderen bevorzugten Ausführungsform wird der Katalysator in einem Katalysatorbett in einem Rohrreaktor angeordnet, wobei der in dieser Ausführungsform insbesondere in Partikeln (bspw. Kugeln) vorliegende Katalysator vorzugsweise im Katalysatorbett fixiert ist, bspw. zwischen Siebplatten angeordnet ist. Unabhängig von der Art des eingesetzten Reaktors wird als in Schritt (I) eingesetzter Katalysator vorzugsweise ein Zeolith-Katalysator verwendet, besonders bevorzugt ein Zeolith vom Typ Y in der protonierten Form (H-Form). Die Anordnung des, insbesondere in Partikelform vorliegenden, Katalysators in einem fixierten Bett ist natürlich nicht auf Rohrreaktoren beschränkt, sondern kann prinzipiell auch bei gerührten Reaktoren zur Anwendung kommen. Ferner ist es möglich, den Katalysator in monolithischer Form einzusetzen.

In der Decarboxylierung von Schritt (I) können bspw. die folgenden Reaktionsparameter eingehalten werden:
- Temperatur bevorzugt im Bereich von 140 °C bis 240 °C und absoluter Druck bevorzugt im Bereich von 1,00 bar bis 20,0 bar,
- Temperatur besonders bevorzugt im Bereich von 160 °C bis 220 °C und absoluter Druck besonders bevorzugt im Bereich von 1,00 bar bis 15,0 bar,
- Temperatur ganz besonders bevorzugt bei einer Temperatur im Bereich von 180 °C bis 200 °C und absoluter Druck ganz besonders bevorzugt im Bereich von 4,00 bar bis 10,0 bar.

Bevorzugt durchläuft der Anilin enthaltende Strom vor seiner Entnahme aus dem Reaktor ein Filter, um zu verhindern, dass Feststoffpartikel (bspw. Katalysatorpartikel) mitgerissen werden.

Schritt (I) wird bevorzugt kontinuierlich durchgeführt, d. h. dass die Edukte (also Aminobenzoesäue und in Schritt (III) zugeführtes Rückführanilin) dem Reaktor kontinuierlich zugeführt und das Produkt (also Rohanilin) dem Reaktor kontinuierlich entnommen wird. In einer Variante dieser Verfahrensweise werden auch mindestens Teile des Katalysators im kontinuierlichen Betrieb permanent oder intervallweise gewechselt, um eine Erschöpfung seiner Leistungsfähigkeit zu verhindern. Eine diskontinuierliche Verfahrensführung (sog. "Batch-Fahrweise") ist aber auch möglich. In einer Variante der diskontinuierlichen Fahrweise (sog. "*Fed-*Batch-Fahrweise") werden die Edukte dem Reaktor so lange kontinuierlich zugeführt, wie es das Reaktorvolumen erlaubt, ohne dass Produkte dem Reaktor entnommen werden. Die Reaktion wird nach Zugabe der maximal möglichen Menge an Edukten unterbrochen und das Produktgemisch dem Reaktor entnommen.

In einer alternativen bevorzugten Ausführungsform ist auch eine Verfahrensführung denkbar, bei welcher die Edukte (also Aminobenzoesäue und in Schritt (III) zugeführtes Rückführanilin) dem Reaktor kontinuierlich zugeführt und das Produkt (also Rohanilin) dem Reaktor kontinuierlich entnommen wird, verbrauchter Katalysator jedoch nicht im kontinuierlichen Betrieb entnommen wird, sondern stattdessen so lange frischer Katalysator (entweder permanent oder intervallweise) zugesetzt wird, bis die durch das vorhandene Reaktorvolumen vorgegebene maximale Katalysatormenge im Reaktor erreicht ist, und dann der Reaktor zwecks Reinigung und Katalysatorwechsel außer Betrieb genommen wird.

In allen Ausführungsformen ist es bevorzugt, Schritt (I) unter Sauerstoffausschluss durchzuführen. Zur Inertisierung des Reaktors eignen sich inerte Gase wie Stickstoff, Kohlenstoffdioxid oder Edelgase.

Erfindungsgemäß ist der Einsatz von systemfremden organischen Lösungsmitteln in Schritt (I) der vorliegenden Erfindung überflüssig. In bevorzugter Ausgestaltung der Erfindung wird daher Schritt (I) in Abwesenheit systemfremder organischer Lösungsmittel durchgeführt. Dies gilt für alle beschriebenen Ausführungsformen des Schrittes (I) und der übrigen Schritte des erfindungsgemäßen Verfahrens.

Der dem Reaktor aus Schritt (I) entnommene Anilin enthaltende Strom wird erfindungsgemäß aufgeteilt. Ein Teil dieses Stroms wird in **Schritt (II)** zur Gewinnung von Anilin aufgereinigt. Diese Aufreinigung kann durch dem Fachmann geläufige Verfahren erfolgen. Insbesondere umfasst die Aufreinigung mindestens einen Destillationsschritt, welchem eine Wasserabtrennung durch Phasentrennung vorgeschaltet sein kann. Die Aufreinigung kann ebenfalls eine Basenbehandlung zur Entfernung saurer Verunreinigungen vor, während oder nach dem Destillationsschritt umfassen. Geeignete Ausgestaltungen sind bspw. in EP-A-1 845 079, EP-A-1 845 080, EP-A-2 263 997 und EP-A-2 028 176 beschrieben. (Diese Schriften befassen sich mit der Reinigung von Anilin, das durch Hydrierung von Nitrobenzol erhalten wurde; die beschriebenen Reinigungsschritte des Rohanilins sind jedoch auf anders hergestelltes Anilin ebenfalls anwendbar.)

Erfindungsgemäß wird ein weiterer Teil des dem Reaktor aus Schritt (I) entnommenen Anilin enthaltenen Stroms (der *Produktstrom* von Schritt (I), sog. "Rohanilin") in den Reaktor aus Schritt (I) zurückgeführt (**Schritt (III)** des erfindungsgemäßen Verfahrens). In einer Ausführungsform der Erfindung wird dem Reaktor aus Schritt (I) nur über diese Rückführung des Anilin enthaltenden Stroms in Schritt (III) Anilin zugeführt.

Unabhängig davon ist es bevorzugt, den dem Reaktor entnommenen Produktstrom nach Abtrennung des für Schritt (II) vorgesehenen Anteils unmittelbar ohne weitere Aufarbeitungsschritte in den Reaktor aus Schritt (I) zurückzuführen. In einer bevorzugten Ausgestaltung der Erfindung wird der dem Reaktor aus Schritt (I) entnommene, Anilin enthaltende Strom in einem Massen-Verhältnis im Bereich von 9,0 : 1 bis 1 : 9,0, bevorzugt im Bereich von 1,5 : 1 bis 1 : 1,5, in zwei Ströme aufgeteilt, von denen einer, bevorzugt der Größere, der Rückführung aus Schritt (III) und der andere der Aufreinigung aus Schritt (II) zugeführt wird.

In einer weiteren bevorzugten Ausgestaltung der Erfindung kann dem Reaktor aus Schritt (I) *zusätzlich* zu dem in Schritt (III) zurückgeführten Anilin enthaltenden Strom ein weiterer Anilinstrom aus einer externen Quelle (bspw. aus dem nach Durchlaufen der Aufreinigung von Schritt (II) erhaltenen Anilinstrom), zugeführt werden. Als zusätzlich zugeführter Anilinstrom eignet sich gereinigtes (vorzugsweise destilliertes) Anilin, und zwar insbesondere Anilin, das einen per Gaschromatographie bestimmten Anilingehalt von mindestens 99,00 Massen-%, bevorzugt mindestens 99,50 Massen-%, besonders bevorzugt mindestens 99,90 Massen-%, bezogen auf die Gesamtmasse des zusätzlich zugeführten Anilinstroms, aufweist. Das auf diese Weise dem Reaktor aus Schritt (I) *zusätzlich* zugeführte Anilin macht nicht mehr als 60 %, bevorzugt von 1,0 % bis 50 %, besonders bevorzugt von 5,0 % bis 20 %, des dem Reaktor aus Schritt (I) *insgesamt* zugeführten Anilins aus. (Der Ausdruck *insgesamt zugeführtes Anilin* bezieht sich hier und im Folgenden auf Anilin *als solches.* Wenn also dem Reaktor in Schritt (III) beispielsweise ein Anilin enthaltender Strom **1** (Rückführanilin) mit *x* kg / h zugeführt wird, wobei der Massenanteil an Anilin in diesem Strom *w₁* beträgt, und dem Reaktor ferner *y* kg / h eines Anilinstroms **2** (zusätzlich zugeführtes gereinigtes Anilin) mit einem Massenanteil an Anilin *w₂* zugeführt werden, so beträgt die dem Reaktor insgesamt pro Stunde zugeführte Masse an Anilin *x* · *w₁* + *y* · *w₂.* Der Massenanteil an Anilin im Rückführanilin ist vom Fachmann einfach zu bestimmen, insbesondere durch High Performance Liquid Chromatographie (HPLC) oder Gaschromatographie, wobei im (unwahrscheinlichen) Falle von signifikanten Abweichungen zwischen einzelnen Bestimmungsmethoden HPLC maßgeblich ist.) Sollte der für die Rückführung in Schritt (III) bestimmte Produktstrom die Decarboxylierung beeinträchtigende Verunreinigungen in einem problematischen Umfang enthalten (feststellbar durch ein Absinken der Anilinkonzentration, gegebenenfalls verbunden mit einen Anstieg der Konzentration an Nebenprodukten), so ist es bevorzugt, den Anteil an zusätzlich zugeführtem gereinigtem (vorzugsweise destilliertem) Anilin (insbesondere Anilin, das die Aufreinigung von Schritt (II) mindestens teilweise - etwa bei einer mehrstufigen Destillation nur die erste Stufe - durchlaufen hat), an dem dem Reaktor aus Schritt (I) *insgesamt* zugeführten Anilin auf einen Anteil von maximal 60 % des dem Reaktor aus Schritt (I) insgesamt zugeführten Anilins zu erhöhen. Es ist daher nicht erforderlich, den dem Reaktor entnommenen Produktstrom vor der Rückführung weiter aufzuarbeiten, weil evtl. störende Verunreinigungen auf diese Weise in einen Bereich unschädlicher Konzentration verdünnt werden können. Diese Maßnahme wird ergriffen, wenn eine zuvor festgelegte Konzentration an Anilin im dem Reaktor aus Schritt (I) entnommenen Anilin enthaltenden Strom unterschritten wird. Die Anilinkonzentration, bezogen auf die Gesamtmasse des Anilin enthaltenden Stroms, der dem Reaktor aus Schritt (I) entnommen wird, kann bevorzugt per HPLC oder Gaschromatographie bestimmt werden, wobei im Zweifelsfall der per Gaschromatographie bestimmte Wert maßgeblich ist. Die Überwachung der Anilinkonzentration kann online oder durch Probennahme in diskreten Zeitintervallen (insbesondere mindestens einmal in 24 Stunden) erfolgen. Der festzulegende Wert für diese bevorzugt nicht zu unterschreitende Anilinkonzentration im dem Reaktor aus Schritt (I) entnommenen Anilin enthaltenden Strom ist abhängig von den genauen Bedingungen in Schritt (I) (die beispielsweise den Wassergehalt des Anilin enthaltenden Stroms maßgeblich beeinflussen) und den Randbedingungen einer vorhandenen Produktionsstätte, insbesondere der Leistungsfähigkeit der für die Aufreinigung in Schritt (II) vorhandenen Einrichtungen (bspw. Destillationskolonnen). Ein allgemeingültiger Wert kann daher nicht angegeben werden, ist aber vom Fachmann durch einfache Vorversuche und/oder Simulationen leicht zu ermitteln.

Die in Schritt (I) zu decarboxylierende Aminobenzoesäure kann grundsätzlich auf jede dem Fachmann bekannte Art gewonnen werden. Eine Möglichkeit ist die Herstellung der Aminobenzoesäure **auf chemischem Wege.** Bevorzugt sind solche Verfahren, die selektiv das ortho-Isomer der Aminobenzoesäure liefern. Als eine geeignete chemische Methode sei beispielhaft die Reaktion von Phthalimid mit Natriumhypochlorit erwähnt. Phthalimid kann seinerseits aus Phthalsäureanhydrid und Ammoniak gewonnen werden. Der ganze Prozess ist wohlbekannt und wird z. B. in Lorz et al., Phthalic Acid and Derivatives in Ullmann's Encyclopedia of Industrial Chemistry, Band 27, S. 140 - 141, Weinheim, Wiley-VCH, beschrieben. Ein industrieller Prozess ist ebenfalls in der Patentliteratur beschrieben; siehe z. B. DE 29 02 978 A1 und EP 0 004 635 A2.

Die Herstellung von para-Aminobenzoesäure auf chemischem Wege kann über die Nitrierung von Toluol mit Salpetersäure, anschließende Oxidation des erhaltenen para-Nitrotoluols mit Sauerstoff zu para-Nitrobenzoesäure und schließlich Reduktion zu para-Aminobenzoesäure mit Hydrazin erfolgen. Der Gesamtprozess ist beispielsweise beschrieben in Maki et al., Benzoic Acid and Derivatives in Ullmann's Encyclopedia of Industrial Chemistry, Band 5, S. 338 ff., Weinheim, Wiley-VCH sowie in O. Kamm, et al. p-Nitrobenzoic acid in Organic Syntheses, Band 1, 1941 S. 392 ff.

Die Herstellung von meta-Aminobenzoesäure gelingt beispielsweise ausgehend von Benzoesäuremethylester: Durch Nitrierung von Benzoesäuremethylester mit Salpetersäure wird meta-Nitrobenzoesäuremethylester erhalten. Dieser Methylester wird anschließend mit Natronlauge verseift. Nach Neutralisation mit Salzsäure wird meta-Nitrobenzoesäure erhalten, die schließlich mit Hydrazin zu meta-Aminobenzoesäure reduziert wird. Das Verfahren wird beispielsweise beschrieben in Maki et al., Benzoic Acid and Derivatives in Ullmann's Encyclopedia of Industrial Chemistry Band 5, S. 338 ff., Weinheim, Wiley-VCH, in Kamm et al, Methyl m-nitrobenzoate in Organic Syntheses, Band 1, 1941, S. 372 ff. sowie in Kamm et al, m-Nitrobenzoic acid in Organic Syntheses. Band 1, 1941, S. 391 ff.

Die in Schritt (I) zu decarboxylierende Aminobenzoesäure wird jedoch **bevorzugt fermentativ** hergestellt. In einer bevorzugten Ausgestaltung der Erfindung wird daher vor Schritt (I) folgender **Schritt (I-0)** durchgeführt, durch welchen die in Schritt (I) zu decarboxylierende Aminobenzoesäure bereitgestellt wird:
(I-0) Fermentation eines Rohstoffes, der wenigstens
- eine fermentierbare Kohlenstoff-haltige Verbindung, bevorzugt ausgewählt aus der Gruppe bestehend aus Stärkehydrolysat, Zuckerrohrsaft, Zuckerrübensaft und Hydrolysaten aus lignocellulosehaltigen Rohstoffen, und
- eine Stickstoff-haltige Verbindung, bevorzugt ausgewählt aus der Gruppe bestehend aus Ammoniakgas, Ammoniakwasser, Ammoniumsalzen (insbesondere Ammoniumsulfat und Ammoniumchlorid) und Harnstoff,
umfasst,
in einem Fermentationsreaktor unter Verwendung von Mikroorganismen unter Erhalt einer Fermentationsbrühe.

Schritt (I-0) des erfindungsgemäßen Verfahrens kann nach einer beliebigen aus dem Stand der Technik bekannten Verfahrensweise durchgeführt werden.

Je nachdem, bei welchem pH-Wert die Fermentation durchgeführt wird, fällt Aminobenzoesäure in Schritt (I-0) nicht in der elektroneutralen Form, sondern z. B. als Aminobenzoat an (für die Art des gebildeten Isomers ist dies jedoch unerheblich). Im Rahmen dieser Erfindung wird im Zusammenhang mit Schritt (I-0) aus Gründen der sprachlichen Vereinfachung regelmäßig von Aminobenzoesäure gesprochen, was so zu verstehen ist, dass die kationische [d. h. diprotonierte], anionische [d. h. deprotonierte] und neutrale [d. h. elektroneutrale] Form von Aminobenzoesäure mit umfasst sind. Wenn jedoch aus den Randbedingungen einer konkret geschilderten Ausführungsform hervorgeht, dass bspw. die deprotonierte Form gebildet wird, so wird von Aminobenzoat gesprochen.

Bevorzugte Mikroorganismen für die Durchführung von Schritt (I-0) sind **Bakterien** oder **Pilze,** und zwar insbesondere **Hefen.** Besonders bevorzugt sind dabei Mikroorganismen einer Art ausgewählt aus der Gruppe bestehend aus *Escherichia coli, Pseudomonas putida, Corynebacterium glutamicum, Ashbya gossypii, Pichia pastoris, Hansenula polymorpha, Yarrowia lipolytica, Zygosaccharomyces bailii und Saccharomyces cerevisiae.* Ganz besonders bevorzugt bestehen die in Schritt (I-0) eingesetzten Mikroorgansimen nur aus Vertretern genau einer dieser Arten, wobei *Corynebacterium glutamicum* ATTC 13032 außerordentlich ganz besonders bevorzugt ist. Der in der Fermentation einzuhaltende pH-Wert richtet sich nach dem eingesetzten Mikroorganismus. Mikroorganismen wie *Corynebacterium glutamicum, Pseudomonas putida* oder *Escherichia coli* werden bevorzugt bei neutralen pH-Werten (d. h. bei einem pH-Wert im Bereich von 6,0 bis 8,0) kultiviert. Mikroorganismen wie *Saccharomyces cerevisiae* werden hingegen bevorzugt im sauren Milieu kultiviert (d. h. bei einem pH-Wert im Bereich von 4,0 bis 5,0).

In jedem Fall wird der Mikroorgansimus aus Schritt (I-0) bevorzugt so ausgewählt, dass in der Fermentation das ortho-Isomer von Aminobenzoesäure gebildet wird.

In einer bevorzugten Ausgestaltung der Erfindung werden **Bakterien als Mikroorganismen** eingesetzt. Dabei wird insbesondere auf die Patentanmeldungen WO 2015/124686 A1 und WO 2015/124687 A1 verwiesen, in denen eine erfindungsgemäß einsetzbare Fermentation unter Einsatz von Bakterien beschrieben ist (siehe beispielsweise WO 2015/124687 A1, (i) Seite 15, Zeile 8 bis Seite 16, Zeile 30, (ii) Beispiel 1 (Seite 29, Zeile 4 bis 26), (iii) Beispiel 3 (vor allem Seite 34, Zeile 10 bis 18), (iv) Beispiel 4 (vor allem Seite 55, Zeile 9 bis 31). Insbesondere kommen Bakterien zum Einsatz, die in der Lage sind, eine fermentierbare Kohlenstoff-haltige Verbindung in Gegenwart einer geeigneten Stickstoffquelle in Aminobenzoesäure umzuwandeln, ohne dass die so gebildete Aminobenzoesäure in zellinternen biochemischen Prozessen gleich wieder verbraucht wird, sodass sich Aminobenzoesäure in der Zelle anreichert und schließlich in die Fermentationsbrühe übergeht.

In einer anderen bevorzugten Ausgestaltung der Erfindung werden **Hefen als Mikroorganismen** eingesetzt. Dabei wird insbesondere auf die noch unveröffentlichte europäische Patentanmeldung mit der Anmeldenummer 16157777.0 verwiesen. Insbesondere kommen Hefezellen zum Einsatz, die in der Lage sind, eine fermentierbare Kohlenstoff-haltige Verbindung in Gegenwart einer geeigneten Stickstoffquelle in Aminobenzoesäure umzuwandeln, ohne dass die so gebildete Aminobenzoesäure in zellinternen biochemischen Prozessen gleich wieder verbraucht wird, sodass sich Aminobenzoesäure in der Zelle anreichert und schließlich in die Fermentationsbrühe übergeht.

Um ein derartiges Bakterium oder eine derartige Hefen zur erhalten, stehen grundsätzlich zwei Wege zur Verfügung, die in bevorzugter Ausgestaltung auch kombiniert werden können:
(i) Die enzymatischen Reaktionen im Aminobenzoesäure-Stoffwechselweg der Bakterienzelle oder Hefezelle können so erhöht werden, dass Aminobenzoesäure schneller produziert als verbraucht wird.
(ii) Die Folgereaktionen, durch welche Aminobenzoesäure in weitere Metaboliten oder Produkte (z. B. Tryptophan) überführt wird, können reduziert bzw. ausgeschaltet werden, sodass sogar die Geschwindigkeit der Aminobenzoesäure-Bildung in Wildtyp-Stämmen ausreichend ist, um zu einer Anreicherung von Aminobenzoesäure in der Zelle zu führen.

Verfahren zur Gewinnung von Bakterien oder Hefezellen mit den zuvor genannten Eigenschaften sind im Stand der Technik bekannt. Geeignete Bakterien oder Hefezellen können beispielsweise durch Screening nach Mutanten, welche Aminobenzoesäure in das umgebende Medium abgeben, identifiziert werden. Die zielgerichtete Modifikation von Schlüsselenzymen mittels gentechnischer Verfahren ist jedoch bevorzugt. Mit üblichen gentechnischen Methoden können Genexpression und Enzymaktivität nach Belieben verstärkt, verringert oder sogar ganz unterbunden werden. Es resultieren rekombinante Stämme.

Besonders bevorzugt enthalten die Bakterien oder Hefezellen, die in der Lage sind, eine fermentierbare Kohlenstoff-haltige Verbindung in Gegenwart einer Stickstoff-haltigen Verbindung zu Aminobenzoesäure umzusetzen, eine Modifizierung der Anthranilat-Phosphoribosyltransferase-Aktivität, welche besagte Enzymaktivität herabsetzt. Durch diese Modifizierung wird die Umwandlung von ortho-Aminobenzoat zu N-(5-Phospho-D-Ribosyl)-Anthranilat reduziert oder komplett unterbunden. Hierdurch wird eine Anreicherung von Aminobenzoesäure in der Zelle bewirkt. Die Bezeichnung "Anthranilat-Phosphoribosyltransferase-Aktivität" bezieht dabei sich auf eine Enzymaktivität, durch welche die Umsetzung von ortho-Aminobenzoat zu N-(5-Phospho-D-Ribosyl)-Anthranilat katalysiert wird.

In Hefen wird die Anthranilat-Phosphoribosyltransferase-Aktivität durch das native Gen TRP4 (YDR354W) genetisch kodiert. In dem Bakterium *Corynebacterium glutamicum* wird die Anthranilat-Phosphoribosyltransferase-Aktivität durch das *trpD* Gen (cg3361, Cg13032, NCg12929) kodiert. Im Falle von *Pseudomonas putida* erfolgt die Kodierung über das *trpD* Gen (PP_0421) innerhalb des *trpDC* Operons.

Die beschriebene Herabsetzung der Anthranilat-Phosphoribosyltransferase-Aktivität kann prinzipiell auf drei Wegen erreicht werden:
(i) Die Regulierung der Expression des Gens für die Anthranilat-Phosphoribosyltransferase-Aktivität kann so modifiziert werden, dass die Transkription des Gens oder anschließende Translation verringert oder unterbunden wird.
(ii) Die Nukleinsäuresequenz des Gens für Anthranilat-Phosphoribosyltransferase-Aktivität kann so modifiziert werden, dass das Enzym, welches durch das modifizierte Gen kodiert wird, eine geringere spezifische Aktivität aufweist.
(iii) Das native Gen für Anthranilat-Phosphoribosyltransferase-Aktivität kann durch ein anderes Gen, das von einem verschiedenen Organismus stammt, ersetzt und ein Enzym mit einer spezifischen Anthranilat-Phosphoribosyltransferase-Aktivität, die geringer ist als die der zuvor erwähnten nativen Gene (z.B. TRP4, *trpD* oder *trpDC*), kodiert werden.

Unabhängig davon, welcher Mikroorganismus eingesetzt wird, umfasst die Fermentationsbrühe zu Beginn der Fermentation in Schritt (I-0) rekombinante Zellen des eingesetzten Mikroorganismus und mindestens eine fermentierbare Kohlenstoff-haltige Verbindung (sowie mindestens eine Stickstoff-haltige Verbindung als Stickstoffquelle). Bevorzugt enthält die Fermentationsbrühe darüber hinaus noch weitere Bestandteile ausgewählt aus der Gruppe bestehend aus Puffersystemen, anorganischen Nährstoffen, Aminosäuren, Vitaminen und weiteren organischen Verbindungen welche für das Wachstum bzw. den Erhaltungsstoffwechsel der rekombinanten Zellen benötigt werden. Die Fermentationsbrühe ist wasserbasiert. Nach Einsetzen des Fermentationsprozesses umfasst die Fermentationsbrühe auch Aminobenzoesäure, das angestrebte Fermentationsprodukt.

Unter einer fermentierbaren Kohlenstoff-haltigen Verbindung im Sinne der vorliegenden Erfindung wird jede organische Verbindung oder Mischung organischer Verbindungen verstanden, die von den rekombinanten Zellen des eingesetzten Mikroorganismus verwendet werden kann, um Aminobenzoesäure zu produzieren. Die Produktion von Aminobenzoesäure kann dabei in Gegenwart oder Abwesenheit von Sauerstoff stattfinden.

Bevorzugt sind dabei solche fermentierbaren Kohlenstoff-haltigen Verbindungen, die zusätzlich als Energie- und Kohlenstoffquelle für das Wachstum der rekombinanten Zellen des eingesetzten Mikroorganismus dienen können. Besonders geeignet sind Stärkehydrolysat, Zuckerrohrsaft, Zuckerrübensaft und Hydrolysate aus lignocellulosehaltigen Rohstoffen. Ebenfalls geeignet sind Glycerin und C1-Verbindungen, insbesondere Kohlenstoffmonoxid.

Erforderlichenfalls wird zwischen der Fermentation in Schritt (I-0) und der Decarboxylierung in Schritt (I) durch pH-Wert-Anpassung sichergestellt, dass Aminobenzoesäure für die Durchführung der Decarboxylierung in der elektroneutralen Form vorliegt. In der Mehrzahl der Fälle ist die Fermentationsbrühe nach Schritt (I-0) basisch bis neutral oder allenfalls leicht sauer (pH > 4,0). Um sicherzustellen, dass die Aminobenzoesäure für die Durchführung der Decarboxylierung in der elektroneutralen Form vorliegt, wird die in Schritt (I-0), gegebenenfalls nach Durchführung von Schritt (α) und/oder Schritt (β), erhaltene Fermentationsbrühe - falls diese Fermentationsbrühe nicht schon hinreichend sauer ist - durch Behandlung mit einer Säure, bevorzugt umfassend Salzsäure, Schwefelsäure und/oder Phosphorsäure, auf einen pH-Wert der resultierenden Mischung im Bereich von 3,0 bis 4,7, bevorzugt im Bereich von 3,2 bis 3,7, besonders bevorzugt im Bereich von 3,4 bis 3,6, eingestellt. In der weiter unten geschilderten Ausführungsform unter Anreicherung der Aminobenzoesäure durch Fällung (Kristallisation) entspricht diese pH-Wert-Anpassung dem Schritt (I-0) **(a)** (i). Sollte die Fermentationsbrühe nach Schritt (I-0) stark sauer (pH < 3,0) sein, wird ein pH-Wert in den vorgenannten Bereichen durch Zugabe von Base (bevorzugt Natronlauge, Kalk) sichergestellt. Liegt der pH-Wert der Fermentationsbrühe nach Schritt (I-0), gegebenenfalls nach Durchführung von Schritt (α) und/oder Schritt (β), im Bereich von 3,0 bis 4,0, wie es beim Einsatz von Hefen als Mikroorganismen der Fall sein kann, wird in einer bevorzugten Ausführungsform weder Säure noch Base zugegeben, sondern die Fermentationsbrühe wird direkt ohne weiter pH-Wert-Anpassung weiter verarbeitet. In diesem Fall ist damit zu rechnen, dass Kristalle von Aminobenzoesäure spontan ausfallen und direkt abgetrennt werden können.

Bevorzugt umfasst der Schritt (I-0) eine *Aufarbeitung der erhaltenen Fermentationsbrühe.* Diese Aufarbeitung, die sich bevorzugt unmittelbar an die eigentliche Fermentation anschließt (die also noch vor der im letzten Absatz geschilderten erforderlichenfalls durchgeführten Säurebehandlung [oder Basenbehandlung] erfolgt), umfasst bevorzugt die folgenden Schritte:
(α) Abtrennung des Mikroorganismus aus der Fermentationsbrühe
   und/oder
(β) Entfärbung der Fermentationsbrühe oder, bei Durchführung von Schritt (α), der in Schritt (a) erhaltenen an Mikroorganismen abgereicherten Fermentationsbrühe.

Die *Abtrennung des Mikroorganismus aus der Fermentationsbrühe* in Schritt (α) ist an sich aus dem Stand der Technik bekannt und erfolgt im Rahmen der vorliegenden Erfindung insbesondere durch Filtration, Absetzen (sog. Settling), Abtrennung in Hydrozyklonen oder Zentrifugation. Eine mögliche Ausgestaltung dieses Schrittes ist in WO 2015/124686 A1 und WO 2015/124687 A1 beschrieben. Dabei wird insbesondere auf WO 2015/124687 A1, Seite 15, Zeile 8 bis Seite 15, Zeile 17, verwiesen.

Unabhängig davon, ob der Mikroorganismus abgetrennt wird oder nicht, kann Schritt (I-0) erforderlichenfalls einen Schritt (β) zur *Entfärbung der Fermentationsbrühe bzw. der an Mikroorganismen abgereicherten Fermentationsbrühe* umfassen. Dieser Schritt (β) wird bevorzugt so durchgeführt, dass Fermentationsbrühe bzw. an Mikroorganismen abgereicherte Fermentationsbrühe über eine Säule mit fester Packung geleitet wird, um Farbstoffe mittels Adsorption zu entfernen. Als mögliche feste Phase kann z. B. Kieselgur oder Ionenaustauscherpackungen verwendet werden. Schritt (β) wird bevorzugt dann durchgeführt, wenn in der Fermentationsbrühe bzw. der an Mikroorganismen abgereicherten Fermentationsbrühe aus Schritt (α) solche farbigen Substanzen vorhanden sind, welche die nachfolgenden Schritte des erfindungsgemäßen Verfahrens, insbesondere die in bevorzugten Ausführungsformen durchgeführte und im Detail noch zu beschreibende *Kristallisation,* stören könnten.

In einer bevorzugten Ausführungsform wird Schritt (I-0) kontinuierlich durchgeführt, d. h. dass die Edukte dem Fermentationsreaktor kontinuierlich zugeführt und das Produkt dem Fermentationsreaktor kontinuierlich entnommen wird. In kontinuierlicher Verfahrensführung wird der Mikroorganismus unter Umständen mit dem Produktstrom ausgetragen; der Mikroorganismus reproduziert sich jedoch im Allgemeinen selbst, sodass eine Zuführung von frischem Mikroorganismus in der Regel nicht nötig ist (erforderlichenfalls aber natürlich vorgenommen werden kann). Eine Zellrückhaltung zur Vermeidung der Austragung von Mikroorganismus ist ebenfalls möglich.

In einer anderen bevorzugten Ausführungsform wird Schritt (I-0) in einer diskontinuierlichen Verfahrensführung (sog. "Batch-Fahrweise") durchgeführt. In einer Variante der diskontinuierlichen Fahrweise (sog. "*Fed*-Batch-Fahrweise") werden die Edukte dem Fermentationsreaktor so lange kontinuierlich zugeführt, wie es das Reaktorvolumen erlaubt, ohne dass Produkte dem Reaktor entnommen werden. Die Reaktion wird nach Zugabe der maximal möglichen Menge an Edukten unterbrochen und das Produktgemisch dem Fermentationsreaktor entnommen.

Unabhängig von der genauen Fahrweise umfasst der Fermentationsreaktor bevorzugt Einrichtungen zur Messungen wichtiger Prozessparameter wie Temperatur, pH-Wert der Fermentationsbrühe, Konzentration an Substrat und Produkt, Gehalt an gelöstem Sauerstoff, Zelldichte der Fermentationsbrühe. Insbesondere bevorzugt umfasst der Fermentationsreaktor Einrichtungen zur Anpassung von mindestens einem (bevorzugt von allen) der vorgenannten Prozessparameter.

Geeignete Fermentationsreaktoren sind Rührkessel, Membranreaktoren, Kolbenstromreaktoren ("plug flow reactors") oder Schlaufenreaktoren (siehe beispielsweise Bioprozesstechnik, Horst Chmiel, ISBN-10: 3827424763, Spektrum Akademischer Verlag). Besonders bevorzugt sowohl für aerobe als auch für anaerobe Fermentationen sind Rührkesselreaktoren und Schlaufenreaktoren (insbesondere Airliftreaktoren, in denen die Zirkulation der Flüssigkeit im Reaktor durch Begasung erreicht wird).

Es hat sich bewährt, nicht die, gegebenenfalls gemäß Schritt (α) und/oder Schritt (β) aufgearbeitete, Fermentationsbrühe unmittelbar zur decarboxylieren (obwohl dies prinzipiell möglich ist), sondern zuvor Aminobenzoesäure in geeigneter Weise anzureichern (**Schritt (I-0) (a)**). Dies kann bspw. durch
(1) Eindampfen der Fermentationsbrühe oder
(2) Fällung in Verbindung mit mindestens teilweiser Abtrennung der abgeschiedenen Aminobenzoesäure von der Mutterlauge
geschehen. Erfindungsgemäß bevorzugt ist die **Variante (2)**. Bei der Fällung wird die, gegebenenfalls wie oben geschildert gemäß Schritt (α) und/oder Schritt (β) aufgearbeitete, Fermentationsbrühe einer Säurebehandlung unterzogen. Bei dieser Säurebehandlung scheidet sich Aminobenzoesäure (bis auf einen der Löslichkeitsgrenze entsprechenden Anteil) ab (*kristallisiert aus*). In dieser Variante (2) umfasst die Säurebehandlung zur Fällung die gegebenenfalls erforderliche oben geschilderte Säurebehandlung zur Überführung der Aminobenzoesäure aus Schritt (I-0) in die elektroneutrale Form. Die abgeschiedene Aminobenzoesäure kann dann abfiltriert und weiter verarbeitet werden. Es ist auch möglich, nur einen Teil der bei der Säurebehandlung (Kristallisation) erhaltenen Mutterlauge abzutrennen und die verbliebene *Suspension an Aminobenzoesäure in Mutterlauge* der Decarboxylierung in Schritt (I) zuzuführen.

In einer besonders bevorzugten Ausgestaltung der Erfindung wird Schritt (I-0) (a) wie folgt durchgeführt:
(i) Behandlung, bevorzugt einstufige Behandlung, der in Schritt (I-0), gegebenenfalls nach Durchführung von Schritt (α) und/oder Schritt (β), erhaltenen Fermentationsbrühe in einem Reaktor mit Säure so, dass sich Aminobenzoesäure aus der Fermentationsbrühe abscheidet, wobei bevorzugt der pH-Wert der resultierenden Mischung auf einen Wert im Bereich von 3,0 bis 4,7, bevorzugt im Bereich von 3,2 bis 3,7, besonders bevorzugt im Bereich von 3,4 bis 3,6, eingestellt wird;
(ii) mindestens teilweise Abtrennung der in Schritt (I-0) (a) (i) abgeschiedenen Aminobenzoesäure von der säurebehandelten Fermentationsbrühe (*der Mutterlauge*);
(iii) optionale weitere Reinigung der in Schritt (I-0) (a) (ii) gewonnenen Aminobenzoesäure, bevorzugt durch Waschen mit Wasser.

In Schritt (1-0) (a) (i) des erfindungsgemäßen Verfahrens wird durch Säurezugabe zur Fermentationsbrühe der pH-Wert so eingestellt, dass Aminobenzoesäure auskristallisiert. Diese Art der Kristallisation wird auch als *Reaktiv*kristallisation bezeichnet. Dies geschieht bevorzugt derart, dass der pH-Wert der resultierenden Mischung dem des isoelektrischen Punkts des abzuscheidenden Isomers von Aminobenzoesäure entspricht oder diesem zumindest nahekommt. Im Falle von ortho-Aminobenzoesäure als gewünschtem Produkt wird daher der pH-Wert bevorzugt auf einen Wert im Bereich von 3,0 bis 4,7, besonders bevorzugt auf einen Wert im Bereich von 3,2 bis 3,7, ganz besonders bevorzugt auf einen Wert im Bereich von 3,4 bis 3,6, eingestellt, also nahe oder entsprechend dem isoelektrischen Punkt bei pH 3,5. Dieser isoelektrische Punkt liegt für die beiden anderen Isomere von Aminobenzoesäure jeweils bei etwa pH 3,7. Die Säurebehandlung in Schritt (I-0) (a) (i) ist dabei bevorzugt "*einstufig*" in dem Sinne, dass der gewünschte Ziel-pH-Wert durch Säurezugabe direkt eingestellt wird, ohne dass bei pH-Werten zwischen dem Ausgangs-pH-Wert (d. i. der pH-Wert der in Schritt (I-0), gegebenenfalls nach Durchführung von Schritt (α) und/oder Schritt (β), erhaltenen Fermentationsbrühe) und dem Ziel-pH-Wert (d. i. der pH-Wert, der sich nach beendeter Säurebehandlung in Schritt (I-0) (a) (i) einstellt) Zwischenschritte (wie Filtration, Zentrifugation, säulenchromatographische Behandlung und dgl.) durchgeführt werden.

Als in Schritt (I-0) (a) (i) einzusetzende Säure kommen alle Säuren in Betracht, mit denen ein pH-Wert, der dem isolektrischen Punkt des gewünschten Aminobenzoesäure-Isomers entspricht oder zumindest nahekommt, eingestellt werden kann. Bevorzugt werden hierzu starke Mineralsäuren, insbesondere Salzsäure, Schwefelsäure und/oder Phosphorsäure eingesetzt. Bevorzugt umfasst die in Schritt (I-0) (a) (i) eingesetzte Säure Salzsäure, besonders bevorzugt Salzsäure einer Konzentration von 15 Massen-% bis 37 Massen-%, ganz besonders bevorzugt Salzsäure einer Konzentration von 18 Massen-% bis 25 Massen-%. Es ist insbesondere bevorzugt, dass die Säure neben dieser Salzsäure mit Ausnahme von optional zugesetzter rezyklierter Mutterlauge aus Schritt (I-0) (a) (ii) keine weitere Säure umfasst (d. h. es wird keine weitere Säure aus einer externen Quelle zugegeben). Wenn als in Schritt (I-0) (a) (i) eingesetzte Säure eine Mischung aus Salzsäure und einem Teil der in Schritt (I-0) (a) (ii) erhaltenen Mutterlauge eingesetzt wird, so werden bevorzugt 1,0 Massen-% bis 50 Massen-% der in Schritt (I-0) (a) (ii) insgesamt erhaltenen Mutterlauge mit Salzsäure vermischt.

Als *Reaktor* in Schritt (I-0) (a) (i) kommen dem Fachmann geläufige übliche Ausgestaltungen chemischer Reaktoren in Betracht. Beispielhaft seien Rührkessel oder Zwangsumlaufkristallisatoren wie solche vom "Typ Oslo" genannt. Die Zugabe der in Schritt (I-0), gegebenenfalls nach Durchführung von Schritt (a) und/oder Schritt (β), erhaltenen Fermentationsbrühe und die Zugabe von Säure in den Reaktor erfolgen bevorzugt kontinuierlich. Das Verfahrensprodukt des Schritts (I-0) (a) (i) - also in sauer gestellter Fermentationsbrühe [= Mutterlauge] suspendierte Aminobenzoesäure - wird dabei dem Reaktor zumindest absatzweise oder bevorzugt ebenfalls kontinuierlich entnommen. In diesem Fall erfolgt vorzugsweise auch die weitere Behandlung in Schritt (I-0) (a) (ii) absatzweise oder kontinuierlich.

Schritt (1-0) (a) (ii), die mindestens partielle Abtrennung der in Schritt (I-0) (a) (i) abgeschiedenen Aminobenzoesäure, ist an sich aus dem Stand der Technik bekannt und erfolgt erfindungsgemäß bevorzugt durch Filtration oder Zentrifugation. Bevorzugt wird dieser Schritt durchgeführt wie in WO 2015/124687 A1 beschrieben. Dabei wird insbesondere auf WO 2015/124687 A1, Seite 17, Zeile 13 bis Seite 17, Zeile 16, verwiesen. Eine Filtration kann bei vermindertem Druck, Umgebungsdruck oder erhöhtem Druck durchgeführt werden. Eine Zentrifugation kann mit handelsüblichen Zentrifugen durchgeführt werden. Es ist auch möglich, die in Schritt (I-0) (a) (i) erhaltene Suspension ruhen zu lassen, bis sich die ausgefallenen Kristalle an Aminobenzoesäure absetzen, und dann die überstehende Mutterlauge abzudekantieren oder abzusaugen. Die verbleibenden, noch mit Mutterlauge benetzten Kristalle an Aminobenzoesäure können Schritt (I) zugeführt werden.

Der optionale Schritt (1-0)(a)(iii), die weitere Reinigung der in Schritt (I-0) (a) (ii) gewonnenen Aminobenzoesäure, ist an sich aus dem Stand der Technik bekannt (siehe vor allem WO 2015/124687 A1 und insbesondere auf WO 2015/124687 A1, Seite 18, Zeile 4 bis Seite 18, Zeile 6) und erfolgt bevorzugt durch eine oder mehrere Wäschen mit wässrigen Waschmedien, insbesondere Wasser. Um Ausbeuteverluste zu vermeiden, kann der pH-Wert des wässrigen Waschmediums auf den gleichen Wert wie in Schritt (I-0) (a) (i) nach beendeter Säurezugabe eingestellt werden; es wird also in dieser Ausführungsform statt mit Wasser mit einer verdünnten Säure, insbesondere der gleichen Säure wie in Schritt (I-0) (a) (i) eingesetzt, gewaschen.

In einer weiteren bevorzugten Ausgestaltung der Erfindung wird der in Schritt (III) in den Reaktor aus Schritt (I) zurückgeführte, Anilin enthaltende Strom mit fester oder gelöster oder suspendierter Aminobenzoesäure versetzt, und zwar bevorzugt in einer solchen Menge, dass der Massenstrom dieser zugeführten festen oder gelösten oder suspendierten Aminobenzoesäure dem Massenstrom des in Schritt (II) der Aufreinigung zugeführten Teils des in Schritt (I) entnommenen, Anilin enthaltenden Stroms entspricht. Diese Ausführungsform ist auf alle Varianten der Erfindung anwendbar, also auch bei chemischer Herstellung der zu decarboxylierenden Aminobenzoesäure. Die fermentative Herstellung der zu decarboxylierenden Aminobenzoesäure ist aber auch hier bevorzugt. Diese Vorgehensweise ermöglicht auf einfache Weise die Zufuhr des Produkts des Fermentationsschrittes in den Decarboxylierungsschritt. Ganz besonders bevorzugt ist es, als Aminobenzoesäure für diesen Zweck eine *aus Schritt (I-0)(a), insbesondere aus Schritt (I-0)(a)(ii) oder aus Schritt (I-0)(a)(iii) stammende* Aminobenzoesäure zu verwenden. Diese Aminobenzoesäure kann vorliegen als
- Suspension (bspw. wenn die Anreicherung der Aminobenzoesäure in Schritt (I-0) (a) durch starkes Eindampfen erfolgt) oder
- Lösung (bspw. wenn die Anreicherung der Aminobenzoesäure in Schritt (I-0) (a) durch geringfügiges Eindampfen erfolgt) oder
- Feststoff (bspw. wenn die Anreicherung der Aminobenzoesäure in Schritt (I-0) (a) durch Säurebehandlung und anschließende Isolierung der ausgefallenen Aminobenzoesäure erfolgt).

In jedem Fall ist besagte Aminobenzoesäure aus Schritt (I-0) (a), insbesondere aus Schritt (I-0) (a) (ii) oder aus Schritt (I-0) (a) (iii) *wasserhaltig.* Im Falle einer Lösung oder Suspension ist dies ohnehin der Fall (da es sich in der Natur der Sache liegend um wässrige Lösungen bzw. Suspensionen handelt). Im Falle eines Feststoffes wird dafür Sorge getragen, dass dieser nicht oder zumindest nicht vollständig getrocknet wird, sondern noch mit Resten an Mutterlauge oder bevorzugt Waschwasser benetzt ist.

In dieser Ausführungsform werden der Wassergehalt und die Menge dieser Aminobenzoesäure vorzugsweise so eingestellt, dass der per Karl-Fischer-Titration bestimmte Wassergehalt der flüssigen Reaktionsmischung in Schritt (I) im Bereich von 0,10 Massen-% bis 40 Massen-%, bevorzugt 0,15 Massen-% bis 20 Massen-%, bezogen auf die Gesamtmasse der flüssigen Reaktionsmischung aus Schritt (I), liegt. In dieser Ausführungsform wird also die in Schritt (I) zu decarboxylierende Aminobenzoesäure im zurückgeführten, Anilin enthaltenden Strom gelöst. Diese Vorgehensweise ist besonders kostengünstig, weil ein zusätzliches systemfremdes Lösungsmittel für die zu decarboxylierende Aminobenzoesäure entfallen kann (was auch die weitere Aufarbeitung vereinfacht, weil eine Trennung von Anilin und systemfremden Lösungsmittel nicht erforderlich ist) und man auf eine weitere Zuführeinrichtung verzichten kann.

Alternativ dazu ist natürlich auch möglich, den in Schritt (III) zurückgeführten, Anilin enthaltenden Strom und die zu decarboxylierende Aminobenzoesäure dem Reaktor aus Schritt (I) *über getrennte Zuführeinrichtungen* (also ohne Vorvermischung) zuzuführen.

Die erfindungsgemäße Vorgehensweise, die Decarboxylierung in einem Reaktionsmedium durchzuführen, das in wesentlichen Anteilen aus zurückgeführtem Rohprodukt (Rückführanilin, enthaltend das rohe, *un*gereinigte Anilin) besteht, hat gegenüber der Variante, nur *gereinigtes* Anilin (und gegebenenfalls systemfremde Lösungsmittel wie 1-Dodecanol) als Lösungsmittel einzusetzen, vielfältige Vorteile:
- Das in Schritt (II) erhaltene Anilin steht, abgesehen von vergleichsweise geringen Anteilen, die optional mit dem in den Reaktor aus Schritt (I) zurückzuführenden, Anilin enthaltenden Strom vermischt werden, unmittelbar für den Verkauf oder die weitere Umsetzung zu Anilinfolgeprodukten zur Verfügung.
- Der Einsatz von Rohanilin ist im Hinblick auf die Betriebskosten (insbesondere Energiekosten) und Investitionskosten (insbesondere was die Anlagengröße angeht) besonders ökonomisch vorteilhaft.
- In bevorzugter Ausgestaltung fallen keinerlei Kosten für systemfremde Lösungsmittel an.

Die erfindungsgemäße Vorgehensweise ermöglicht des Weiteren im Vergleich zu der aus dem Stand der Technik bekannten Decarboxylierung in 1-Dodecanol den problemlosen Einsatz von feuchtem Edukt (Aminobenzoesäure), insbesondere von, bezogen auf die Gesamtmasse des einzusetzenden Edukts, bis zu 40 Massen-% Wasser enthaltendem Edukt als Ausgansmaterial, was sich positiv auf die Betriebs- und Investitionskosten des in bevorzugten Ausgestaltungen eingesetzten Kristallisationsschrittes [Schritt (I-0) (a) (i) bis (ii) bzw. (i) bis (iii)] auswirkt, da ein Trocknungsschritt entfallen oder zumindest einfacher ausgestaltet werden kann.

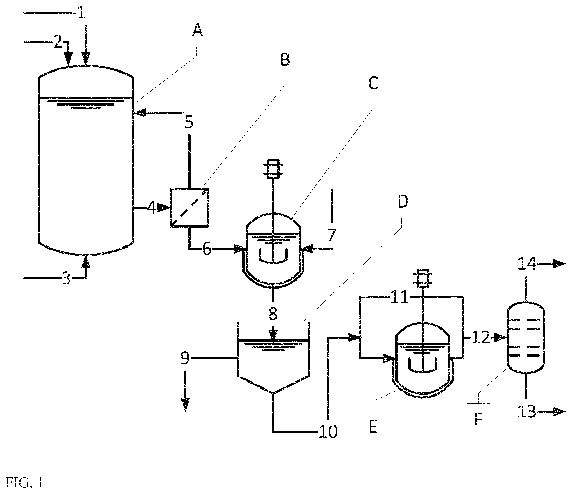

FIG. 1 zeigt eine bevorzugte Ausgestaltung des erfindungsgemäßen Verfahrens.

### Bezugszeichenliste:

### Apparate:

A) Fermentationsreaktor
B) Zelltrennung
C) Reaktor für Kristallisation
D) Filter/Dekanter
E) Reaktor für die Decarboxylierung
F) Destillationskolonne

### Stoffströme:

1) Fermentierbare Kohlenstoff-haltige Verbindung
2) Stickstoff-haltige Verbindung
3) Sauerstoff- oder Luftquelle für den Fermentationsreaktor im Falle von aeroben Fermentationen
4) Fermentationsbrühe mit Mikroorganismen
5) In den Fermentationsreaktor zurückgeführte Mikroorganismen
6) An Mikroorganismen abgereicherte (insbesondere von Mikroorganismen befreite) Fermentationsbrühe
7) Säure (insbesondere Salzsäure) zum Ausfällen von Aminobenzoesäure
8) Suspension an Aminobenzoesäure
9) Mutterlauge zur weiteren Aufarbeitung
10) Suspension an Aminobenzoesäure-Kristallen
11) Rohanilin-Rückführung
12) Rohanilinstrom zur Destillation
13) Sumpfstrom der Anilindestillation (überwiegend hochsiedende Nebenprodukte)
14) Gereinigtes Anilin zur weiteren Verwendung

Das erfindungsgemäß gewonnene Anilin wird bevorzugt zu einem *Anilinfolgeprodukt* weiter umgesetzt, d. h. **Schritt (IV)** wird bevorzugt durchgeführt. Ausgewählte weitere Umsetzungen des erhaltenen Anilins in Schritt (IV) sind:
(IV-1) säurekatalysierte Reaktion des Anilins mit Formaldehyd unter Bildung von Di- und Polyaminen der Diphenylmethanreihe;
(IV-2) säurekatalysierte Reaktion des Anilins mit Formaldehyd, gefolgt von Umsetzung mit Phosgen unter Bildung von Di- und Polyisocyanaten der Diphenylmethanreihe;
(IV-3) Umsetzung des Anilins zu einer Azoverbindung.

Die weitere Umsetzung Anilins mit Formaldehyd zu **Di- und Polyaminen der Diphenylmethanreihe (IV-1)** ist an sich bekannt und kann nach einem beliebigen Verfahren des Standes der Technik durchgeführt werden. Die kontinuierliche oder teilweise diskontinuierliche Herstellung von Di- und Polyaminen der Diphenylmethanreihe aus Anilin und Formaldehyd ist z. B. in EP 1 616 890 A1, US-A-5286760, EP-A-451442 und WO-A-99/40059 offenbart. Die Reaktion erfolgt unter Säurekatalyse. Als saurer Katalysator eignet sich vorzugsweise Salzsäure.

Die weitere Umsetzung der so erhaltenen Di- und Polyamine der Diphenylmethanreihe mit Phosgen zu **Di- und Polyisocyanaten der Diphenylmethanreihe** (**IV-2**) ist ebenfalls an sich bekannt und kann nach einem beliebigen Verfahren des Standes der Technik durchgeführt werden. Geeignete Verfahren sind beispielsweise in EP 2 077 150 B1, EP 1 616 857 A1, EP 1 873 142 A1, und EP 0 314 985 B1 beschrieben.

Die Umsetzung des erfindungsgemäß erhaltenen Anilins zu **Azoverbindungen, insbesondere zu Azo-Farbstoffen** (**IV-3**) kann nach einem beliebigen Verfahren des Standes der Technik erfolgen. Beispielhaft sei auf die bekannte Herstellung von Anilingelb (para-Aminoazobenzol; CAS 493-5-7) oder Indigo (2,2'-Bis(2,3-dihydro-3-oxomethyliden); CAS 482-89-3) verwiesen (Per Wiklund et al., Current Organic Synthesis, 2006, 3, 379 - 402).

### Beispiele

In allen Beispielen wurde die jeweils eingesetzte ortho-Aminobenzoesäure (oAB) hergestellt durch Fermentation von rekombinanten *Corynebacterium-glutamicum*-ATTC-13032-Stämmen, die eine Deletion bzw. reduzierte Expression des trpD Gens aufweisen, welches für die Anthranilat-Phosphoribosyltransferase kodiert wie in WO 2015/124687 A1, Beispiel 3 beschrieben (entspricht **Schritt (I-0)** des erfindungsgemäßen Verfahrens). Zur Abreicherung des eingesetzten Mikroorganismus wurde die Fermentationsbrühe filtriert (entspricht **Schritt (I-0) (α)** des erfindungsgemäßen Verfahrens). Die oAB wurde aus der Fermentationsbrühe mittels Kristallisation durch Zugabe von Salzsäure, Filtration und Waschen mit Wasser isoliert (entspricht **Schritt (I-0) (a), Variante (2)** des erfindungsgemäßen Verfahrens).

### Beispiel la (Vergleichsbeispiel)

Ortho-Aminobenzoesäure (oAB) wird so in Anilin (Sigma Aldrich, > 99 % Reinheit) gelöst, dass eine Lösung mit einem Massenanteil an oAB von 40 % erhalten wird. Man erhält bei Raumtemperatur eine viskose Suspension, die bei Temperaturerhöhung auf 60 bis 80 °C in Lösung geht. 100 g dieser Lösung werden in einen Autoklaven als Reaktor überführt, in dem sich 8,00 g Zeolith-Katalysator H-Y (CBV-600 von *Zeolyst International,* zur Entfernung von Feuchtigkeit gemäß Herstellerangaben vorbehandelt) befinden. Der Reaktor wird verschlossen und mit Ar gespült, um Sauerstoff auszutreiben. Die Reaktionsmischung wird auf 180 °C erhitzt. Diese Temperatur wird 1,0 h gehalten. Der Druckanstieg (infolge CO₂-Entwicklung) wird gemessen. Ebenso werden in 10-minütigem Abstand Proben gezogen und mittels HPLC analysiert. Nach 40 Minuten konnte kein weiterer Druckanstieg festgestellt werden; oAB war zu mehr als 99 % umgesetzt worden. Die Reaktion ergab mit einer Selektivität von > 98 % Anilin.

### Beispiel 1b (Vergleichsbeispiel)

Beispiel 1a wurde in Gegenwart von 10 Massen-% Wasser wiederholt. Die Ergebnisse sind ähnlich wie bei Beispiel 1a. Nach 40 Minuten war oAB auch zu mehr als 99 % umgesetzt worden. Die Reaktion ergab mit einer Selektivität von > 98 % Anilin.

### Beispiel 2 (erfindungsgemäß)

Nach Abkühlung wird das Rohprodukt (86 g) der Reaktion aus Beispiel 1a über eine mit einem Filter versehene Leitung dem Reaktor entnommen. 60 g dieses Produkts werden mit 40 g oAB vermischt (Massenanteil von oAB 40 %) und erneut in den Reaktor eingeführt. Es werden die gleichen Reaktionsbedingungen wie in Beispiel 1a eingehalten. Der Katalysator wird nicht gewechselt. Es werden insgesamt 10 Reaktionszyklen durchgeführt. Der Umsatz an oAB betrug im ersten Reaktionszyklus 98,8% und stabilisierte sich in den folgenden Reaktionszyklen auf Werte zwischen 97,7 % und 98,2 %.

Beispiel 2 zeigt, dass der Katalysator trotz der 10-maligen Rezyklierung von Rohanilin weiterhin verlässlich mit hoher Aktivität und Selektivität Anilin produziert. Dies war völlig überraschend; es wäre zu erwarten gewesen, dass die Verunreinigungen des Rohanilinstroms den Katalysator deaktivieren.

## Patentansprüche

1. Verfahren Herstellung von Anilin oder eines Anilinfolgeprodukts, wobei das Verfahren die folgenden Schritte umfasst:
(I) Decarboxylierung von Aminobenzoesäure zu Anilin in einem Reaktor in Gegenwart eines Katalysators, wobei ein Anilin enthaltender Strom dem Reaktor entnommen wird;
(II) Aufreinigung eines Teils des in Schritt (I) entnommenen, Anilin enthaltenden Stroms zur Gewinnung von Anilin, bevorzugt durch Destillation;
(III) Rückführung eines anderen Teils des in Schritt (I) entnommenen, Anilin enthaltenden Stroms in den Reaktor aus Schritt (I);
(IV) optionale weitere Umsetzung des in Schritt (II) aufgereinigten Anilins zu einem Anilinfolgeprodukt, wobei Schritt (IV) insbesondere eine der folgenden Umsetzungen umfasst:
(IV-1) säurekatalysierte Reaktion des Anilins mit Formaldehyd unter Bildung von Di- und Polyaminen der Diphenylmethanreihe;
(IV-2) säurekatalysierte Reaktion des Anilins mit Formaldehyd, gefolgt von Umsetzung mit Phosgen unter Bildung von Di- und Polyisocyanaten der Diphenylmethanreihe;
(IV-3) Umsetzung des Anilins zu einer Azoverbindung.

2. Verfahren gemäß Anspruch 1, bei welchem dem Reaktor aus Schritt (I) zusätzlich zu dem in Schritt (III) zurückgeführten Anilin enthaltenden Strom ein weiterer Anilinstrom aus gereinigtem Anilin zugeführt wird, wobei das auf diese Weise dem Reaktor aus Schritt (I) zusätzlich zugeführte Anilin maximal 60 % des dem Reaktor aus Schritt (I) insgesamt zugeführten Anilins ausmacht.

3. Verfahren gemäß Anspruch 2, bei welchem die Konzentration an Anilin in dem Anilin enthaltenden Strom, der dem Reaktor aus Schritt (I) entnommen wird, überwacht wird, und bei festgestellter Unterschreitung eines zuvor festgelegten Werts der Konzentration an Anilin der Anteil des dem Reaktor zusätzlich zugeführten Anilinstroms aus gereinigtem Anilin auf einen Wert von maximal 60 % des dem Reaktor aus Schritt (I) insgesamt zugeführten Anilins erhöht wird.

4. Verfahren gemäß einem der vorstehenden Ansprüche, bei welchem der dem Reaktor aus Schritt (I) entnommene, Anilin enthaltende Strom in einem Massen-Verhältnis im Bereich von 9,0 : 1 bis 1 : 9,0 in zwei Ströme aufgeteilt wird, von denen einer der Rückführung aus Schritt (III) und der andere der Aufreinigung aus Schritt (II) zugeführt wird.

5. Verfahren gemäß einem der vorstehenden Ansprüche, bei dem Schritt (I) bei einer Temperatur im Bereich von 140 °C bis 240 °C und bei einem absoluten Druck im Bereich von 1,00 bar bis 20,0 bar durchgeführt wird.

6. Verfahren gemäß einem der vorstehenden Ansprüche, bei welchem der Reaktor aus Schritt (I)
ein Schlammphasenreaktor ist, und der Katalysator in einer Konzentration im Bereich von 0,100 Massen-% bis 50,0 Massen-% bezogen auf die Gesamtmasse der flüssigen Reaktionsmischung, eingesetzt wird
oder
ein Rührkesselreaktor ist,
oder
ein Rohrreaktor mit einem Katalysatorbett ist.

7. Verfahren gemäß einem der vorstehenden Ansprüche, bei welchem der in Schritt (I) eingesetzte Katalysator ein Zeolith-Katalysator ist.

8. Verfahren gemäß einem der vorstehenden Ansprüche, bei welchem die in Schritt (I) zu decarboxylierende Aminobenzoesäure durch folgenden, vor Schritt (I) durchzuführenden Schritt (I-0) bereitgestellt wird:
(I-0) Fermentation eines Rohstoffes, der wenigstens
• eine fermentierbare Kohlenstoff-haltige Verbindung und
• eine Stickstoff-haltige Verbindung
umfasst,
in einem Fermentationsreaktor unter Verwendung von Mikroorganismen unter Erhalt einer Fermentationsbrühe; woran sich optional die folgenden Aufarbeitungsschritte anschließen:
(α) Abtrennung des Mikroorganismus aus der Fermentationsbrühe und/oder
(β) Entfärbung der Fermentationsbrühe oder, bei Durchführung von Schritt (α), der in Schritt (α) erhaltenen an Mikroorganismen abgereicherten Fermentationsbrühe.

9. Verfahren gemäß Anspruch 8, bei welchem nach Schritt (I-0) und vor Schritt (I) folgender Schritt durchgeführt wird
(I-0) (a) Anreicherung der Aminobenzoesäure durch eine der folgenden Maßnahmen:
(1) Eindampfen der Fermentationsbrühe, oder
(2) Fällung durch Säurebehandlung in Verbindung mit mindestens teilweiser Abtrennung der abgeschiedenen Aminobenzoesäure von der säurebehandelten Fermentationsbrühe.

10. Verfahren gemäß Anspruch 9, bei welchem Schritt (I-0) (a) gemäß Variante (2) durchgeführt wird und Folgendes umfasst:
(i) Behandlung der in Schritt (I-0), gegebenenfalls nach Durchführung von Schritt (α) und/oder Schritt (β), erhaltenen Fermentationsbrühe in einem Reaktor mit Säure so, dass sich Aminobenzoesäure aus der Fermentationsbrühe abscheidet;
(ii) mindestens teilweise Abtrennung der in Schritt (I-0) (a) (i) abgeschiedenen Aminobenzoesäure von der säurebehandelten Fermentationsbrühe;
(iii) optionale weitere Reinigung der in Schritt (I-0) (a) (ii) gewonnenen Aminobenzoesäure.

11. Verfahren gemäß Anspruch 10, bei welchem die in Schritt (I-0) (a) (i) eingesetzte Säure Salzsäure, Schwefelsäure und/oder Phosphorsäure umfasst.

12. Verfahren gemäß einem der Ansprüche 8 bis 11, bei welchem die in Schritt (I-0) eingesetzten Mikroorganismen eine Art ausgewählt aus der Gruppe bestehend aus *Escherichia coli, Pseudomonas putida, Corynebacterium glutamicum, Ashbya gossypii, Pichia pastoris, Hansenula polymorpha, Yarrowia lipolytica, Zygosaccharomyces bailii und Saccharomyces cerevisiae,* umfassen.

13. Verfahren gemäß einem der vorstehenden Ansprüche, insbesondere gemäß einem der Ansprüche 8 bis 12, bei welchem der in Schritt (III) in den Reaktor aus Schritt (I) zurückgeführte, Anilin enthaltende Strom mit fester oder gelöster oder suspendierter Aminobenzoesäure versetzt wird.

14. Verfahren gemäß Anspruch 13, insofern dieser auf einen der Ansprüche 9 bis 12 Bezug nimmt, bei welchem der in Schritt (III) in den Reaktor aus Schritt (I) zurückgeführte, Anilin enthaltende Strom mit fester oder gelöster oder suspendierter Aminobenzoesäure versetzt wird, wobei diese aus Schritt (I-0) (a), insbesondere aus Schritt (I-0) (a) (ii) oder aus Schritt (I-0) (a) (iii) stammt und wasserhaltig ist.

15. Verfahren gemäß einem der Ansprüche 1 bis 12, insbesondere gemäß einem der Ansprüche 8 bis 12, bei welchem der in Schritt (III) zurückgeführte, Anilin enthaltende Strom und die zu decarboxylierende Aminobenzoesäure dem Reaktor aus Schritt (I) über getrennte Zuführeinrichtungen zugeführt werden.

## Claims

1. Method for producing aniline or an aniline conversion product, wherein the method comprises the following steps:
(I) decarboxylating aminobenzoic acid to aniline in a reactor in the presence of a catalyst, wherein a stream containing aniline is withdrawn from the reactor;
(II) purifying a portion of the stream containing aniline withdrawn in step (I) to obtain aniline, preferably by distillation;
(III) recirculating another portion of the stream containing aniline withdrawn in step (I) into the reactor of step (I);
(IV) optionally further reacting the aniline purified in step (II) to give an aniline conversion product, wherein step (IV) particularly includes one of the following reactions:
(IV-1) acid-catalyzed reaction of aniline with formaldehyde to form di- and polyamines of the diphenylmethane series;
(IV-2) acid-catalyzed reaction of aniline with formaldehyde, followed by reaction with phosgene to form di- and polyisocyanates of the diphenylmethane series;
(IV-3) reacting aniline to give an azo compound.

2. Method according to Claim 1, in which a further aniline stream of purified aniline is fed to the reactor of step (I) in addition to the stream containing aniline recirculated in step (III), wherein the aniline additionally fed in this manner to the reactor of step (I) accounts for at most 60% of the total aniline fed to the reactor of step (I).

3. Method according to Claim 2, in which the concentration of aniline is monitored in the stream comprising aniline which is withdrawn from the reactor of step (I), and when a shortfall in the concentration of aniline of a previously set value is detected, the proportion of the aniline stream of purified aniline additionally fed to the reactor is increased to a value of at most 60% of the aniline fed in total to the reactor of step (I).

4. Method according to any of the preceding claims, in which the stream containing aniline withdrawn from the reactor of step (I) is divided into two streams in a ratio by mass in the range from 9.0:1 to 1:9.0, of which one is fed to the recirculation of step (III) and the other to the purification of step (II).

5. Method according to any of the preceding claims, in which step (I) is conducted at a temperature in the range from 140°C to 240°C and at an absolute pressure in the range from 1.00 bar to 20.0 bar.

6. Method according to any of the preceding claims, in which the reactor of step (I)
is a slurry phase reactor, and the catalyst is used at a concentration in the range from 0.100% by mass to 50.0% by mass, based on the total mass of the liquid reaction mixture
or
is a stirred tank reactor,
or
is a tubular reactor having a catalyst bed.

7. Method according to any of the preceding claims, in which the catalyst used in step (I) is a zeolite catalyst.

8. Method according to any of the preceding claims, in which the aminobenzoic acid to be decarboxylated in step (I) is provided by the following step (I-0) to be carried out prior to step (I):
(I-0) fermentation of a raw material, which comprises at least
• one fermentable carbon-containing compound and
• one nitrogen-containing compound,
in a fermentation reactor using microorganisms to obtain a fermentation broth; which is then optionally followed by the following work-up steps:
(α) removing the microorganism from the fermentation broth
and/or
(β) decolorizing the fermentation broth or, in the case of step (α) being carried out, the fermentation broth depleted of microorganisms obtained in step (α).

9. Method according to Claim 8, in which the following step is carried out after step (I-0) and prior to step (I):
(I-0) (a) enrichment of the aminobenzoic acid by one of the following measures:
(1) evaporation of the fermentation broth, or
(2) precipitation by acid treatment combined with at least partial removal of the aminobenzoic acid that separates out from the acid-treated fermentation broth.

10. Method according to Claim 9, in which step (I-0) (a) is carried out according to variant (2) and comprises the following:
(i) treating the fermentation broth obtained in step (I-0), optionally after carrying out step (α) and/or step (β), in a reactor with acid such that aminobenzoic acid separates out from the fermentation broth;
(ii) at least partially removing the aminobenzoic acid that separates out in step (I-0) (a) (i) from the acid-treated fermentation broth;
(iii) optional further purification of the aminobenzoic acid obtained in step (I-0) (a) (ii).

11. Method according to Claim 10, in which the acid used in step (I-0) (a) (i) comprises hydrochloric acid, sulfuric acid and/or phosphoric acid.

12. Method according to any of Claims 8 to 11, in which the microorganisms used in step (I-0) comprise a species selected from the group consisting of *Escherichia coli, Pseudomonas putida, Corynebacterium glutamicum, Ashbya gossypii, Pichia pastoris, Hansenula polymorpha, Yarrowia lipolytica, Zygosaccharomyces bailii* and *Saccharomyces cerevisiae.*

13. Method according to any of the preceding claims, especially according to any of Claims 8 to 12, in which solid or dissolved or suspended aminobenzoic acid is added to the stream containing aniline recirculated in step (III) into the reactor of step (I).

14. Method according to Claim 13, insofar as this refers to any of Claims 9 to 12, in which to the stream containing aniline recirculated in step (III) into the reactor of step (I) is added solid or dissolved or suspended aminobenzoic acid, wherein this originates from step (I-0) (a), especially from step (I-0) (a) (ii) or from step (I-0) (a) (iii), and contains water.

15. Method according to any of Claims 1 to 12, especially according to any of Claims 8 to 12, in which the stream containing aniline recirculated in step (III) and the aminobenzoic acid to be decarboxylated are fed to the reactor of step (I) via separate feed units.

## Revendications

1. Procédé de fabrication d'aniline ou d'un produit dérivé de l'aniline, le procédé comprenant les étapes suivantes :
(I) la décarboxylation d'acide aminobenzoïque en aniline dans un réacteur en présence d'un catalyseur, un courant contenant de l'aniline étant soutiré du réacteur ;
(II) la purification d'une partie du courant contenant de l'aniline soutiré à l'étape (I) pour obtenir de l'aniline, de préférence par distillation ;
(III) le recyclage d'une autre partie du courant contenant de l'aniline soutiré à l'étape (I) dans le réacteur de l'étape (I) ;
(IV) éventuellement la mise en réaction supplémentaire de l'aniline purifiée à l'étape (II) pour former un produit dérivé de l'aniline, l'étape (IV) comprenant notamment une des réactions suivantes :
(IV-1) une réaction sous catalyse acide de l'aniline avec du formaldéhyde avec formation de di- et polyamines de la série du diphénylméthane ;
(IV-2) une réaction sous catalyse acide de l'aniline avec du formaldéhyde, suivie par une réaction avec du phosgène avec formation de di- et polyisocyanates de la série du diphénylméthane ;
(IV-3) une réaction de l'aniline pour former un composé azo.

2. Procédé selon la revendication 1, selon lequel un courant d'aniline supplémentaire constitué par de l'aniline purifiée est introduit dans le réacteur de l'étape (I) en plus du courant contenant de l'aniline recyclé à l'étape (III), l'aniline introduite en outre dans le réacteur de l'étape (I) de cette manière représentant au plus 60 % de l'aniline introduite au total dans le réacteur de l'étape (I).

3. Procédé selon la revendication 2, selon lequel la concentration en aniline dans le courant contenant de l'aniline qui est soutiré du réacteur de l'étape (I) est surveillée et, s'il est observé que la concentration en aniline passe en dessous d'une valeur fixée au préalable, la proportion du courant d'aniline constitué par de l'aniline purifiée introduit en outre dans le réacteur est augmentée à une valeur d'au plus 60 % de l'aniline introduite au total dans le réacteur de l'étape (I).

4. Procédé selon l'une quelconque des revendications précédentes, selon lequel le courant contenant de l'aniline soutiré du réacteur de l'étape (I) est divisé en deux courants en un rapport en masse dans la plage allant de 9,0:1 à 1:9,0, parmi lesquels un est acheminé dans le recyclage de l'étape (III) et l'autre dans la purification de l'étape (II).

5. Procédé selon l'une quelconque des revendications précédentes, selon lequel l'étape (I) est réalisée à une température dans la plage allant de 140 °C à 240 °C et à une pression absolue dans la plage allant de 1,00 bar à 20,0 bar.

6. Procédé selon l'une quelconque des revendications précédentes, selon lequel le réacteur de l'étape (I) est un réacteur à phase suspension, et le catalyseur est utilisé en une concentration dans la plage allant de 0,100 % en masse à 50,0 % en masse, par rapport à la masse totale du mélange réactionnel liquide,
ou
est un réacteur à cuve agitée,
ou
est un réacteur tubulaire contenant un lit catalytique.

7. Procédé selon l'une quelconque des revendications précédentes, selon lequel le catalyseur utilisé à l'étape (I) est un catalyseur à base de zéolithe.

8. Procédé selon l'une quelconque des revendications précédentes, selon lequel l'acide aminobenzoïque à décarboxyler à l'étape (I) est préparé par l'étape (I-0) suivante, à réaliser avant l'étape (I) :
(I-0) la fermentation d'une matière première, qui comprend au moins :
- un composé carboné fermentable et
- un composé azoté,
dans un réacteur de fermentation en utilisant des microorganismes avec obtention d'un bouillon de fermentation ; les étapes de traitement suivantes s'ensuivant éventuellement :
(α) la séparation du microorganisme du bouillon de fermentation
et/ou
(β) la décoloration du bouillon de fermentation ou, lors de la réalisation de l'étape (α), du bouillon de fermentation appauvri en microorganismes obtenu à l'étape (α).

9. Procédé selon la revendication 8, selon lequel l'étape suivante est réalisée après l'étape (I-0) et avant l'étape (I) :
(I-0) (a) la concentration de l'acide aminobenzoïque par une des mesures suivantes :
(1) une évaporation du bouillon de fermentation, ou
(2) une précipitation par traitement acide conjointement avec une séparation au moins partielle de l'acide aminobenzoïque isolé du bouillon de fermentation traité par un acide.

10. Procédé selon la revendication 9, selon lequel l'étape (I-0) (a) est réalisée selon la variante (2) et comprend les étapes suivantes :
(i) le traitement du bouillon de fermentation obtenu à l'étape (I-0), éventuellement après la réalisation de l'étape (α) et/ou de l'étape (β), dans un réacteur avec un acide, de telle sorte que l'acide aminobenzoïque soit isolé du bouillon de fermentation ;
(ii) la séparation au moins partielle de l'acide aminobenzoïque isolé à l'étape (I-0) (a) (i) du bouillon de fermentation traité avec un acide ;
(iii) la purification supplémentaire éventuelle de l'acide aminobenzoïque obtenu à l'étape (I-0) (a) (ii).

11. Procédé selon la revendication 10, selon lequel l'acide utilisé à l'étape (I-0) (a) (i) est l'acide chlorhydrique, l'acide sulfurique et/ou l'acide phosphorique.

12. Procédé selon l'une quelconque des revendications 8 à 11, selon lequel les microorganismes utilisés à l'étape (I-0) comprennent une espèce choisie dans le groupe constitué par *Escherichia coli, Pseudomonas putida, Corynebacterium glutamicum, Ashbya gossypii, Pichia pastoris, Hansenula polymorpha, Yarrowia lipolytica, Zygosaccharomyces bailii* et *Saccharomyces cerevisiae.*

13. Procédé selon l'une quelconque des revendications précédentes, notamment selon l'une quelconque des revendications 8 à 12, selon lequel le courant contenant de l'aniline recyclé dans le réacteur de l'étape (I) à l'étape (III) est mélangé avec de l'acide aminobenzoïque solide ou dissous ou suspendu.

14. Procédé selon la revendication 13, dans la mesure où celui-ci se rapporte à l'une quelconque des revendications 9 à 12, selon lequel le courant contenant de l'aniline recyclé dans le réacteur de l'étape (I) à l'étape (III) est mélangé avec de l'acide aminobenzoïque solide ou dissous ou suspendu, celui-ci provenant de l'étape (I-0) (a), notamment de l'étape (I-0) (a) (ii) ou de l'étape (I-0) (a) (iii), et contenant de l'eau.

15. Procédé selon l'une quelconque des revendications 1 à 12, notamment selon l'une quelconque des revendications 8 à 12, selon lequel le courant contenant de l'aniline recyclé à l'étape (III) et l'acide aminobenzoïque à décarboxyler sont introduits dans le réacteur de l'étape (I) par des dispositifs d'introduction séparés.
